(19) Europäisches Patentamt
European Patent Office
Office européen des brevets

(11) **EP 4 613 193 A1**

(12) **EUROPEAN PATENT APPLICATION**

(43) Date of publication:
**10.09.2025 Bulletin 2025/37**

(21) Application number: **25161525.8**

(22) Date of filing: **04.03.2025**

(51) International Patent Classification (IPC):
**A61B 5/145** (2006.01)   **A61B 5/1477** (2006.01)
**A61B 5/1486** (2006.01)   **A61B 5/15** (2006.01)
**A61B 5/00** (2006.01)

(52) Cooperative Patent Classification (CPC):
**A61B 5/14532; A61B 5/14514; A61B 5/14546;**
**A61B 5/1477; A61B 5/1486; A61B 5/150022;**
**A61B 5/150076; A61B 5/6833; A61B 10/0045;**
A61B 2010/008; A61B 2562/125

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **04.03.2024 US 202418594175**

(71) Applicant: **Cambridge Medical Technologies LLC**
**Carson City, Nevada 89701 (US)**

(72) Inventors:
• **JACHMANN, Emil F.**
**New Canaan, 06840 (US)**

• **CURRIE, John Frederick**
**Bethesda, 20817 (US)**
• **BHATIA, Vikas**
**Gaithersburg, 20882 (US)**
• **WENSMAN, John**
**Washington, 20007 (US)**
• **CURRIE, Georgia**
**Bethesda, 20817 (US)**

(74) Representative: **Kompter, Hans-Michael**
**Heumann**
**Rechtsanwälte- und Patentanwälte**
**Spessartring 63**
**64287 Darmstadt (DE)**

(54) **NON-INVASIVE TRANSDERMAL SAMPLING AND ANALYSIS DEVICE FOR DETECTION OF MULTIPLE ANALYTES**

(57)   Transdermal sampling and analysis devices, methods, and systems are provided. The transdermal sampling and analysis device may include a base structure with a disruptor and a first electrode array, a lid structure with a second electrode array, and an adhesive structure containing a hole to direct interstitial fluid to at least one of the first and second electrode arrays. The device may also include at least one conductive trace configured for external connection to one or more of a voltage/current source and a current detector, and an electrically conductive material that passes through a hole in the adhesive layer, providing a conductive path between the base structure and the lid structure. Methods and systems for manufacturing a plurality of biosensing chips that may be used in the transdermal sampling and analysis devices are also provided.

FIG. 3

EP 4 613 193 A1

**Description**

**RELATED APPLICATIONS**

This European patent application claims the priority of U.S. Patent Application No. 18/594,175, entitled "Non-Invasive Transdermal Sampling and Analysis Device for Detection of Multiple Analytes, "filed on March 4, 2024, which is incorporated by reference in its entirety herein for all purposes.

**BACKGROUND**

A biosensor is a device that may measure the presence or level of an analyte in a biological sample. A biosensor may include three main parts: i) biologically reactive elements sometimes referred to as reagents. Reagents may include biological materials (e.g., tissues, microorganisms, organelles, cell receptors, enzyme, antibodies, and take acid, etc.), a biologically derived material or biomimic, wherein the reagent may be created by biological engineering; ii) a transducer or detector element which may work in a physiochemical way (e.g., optical, piezoelectric, electrochemical, etc.) to transform the signal resulting from the interaction of the analyte being measured with the reagent into another signal that may be more easily measured and quantified; and iii) associated electronics and/or signal processors that may be primarily responsible for the display of the results.

Enzymatic amperometric biosensors involve placement of an enzyme in close proximity to an electrode surface. The enzyme involved may catalyze the reaction, which involves consuming of electroactive reactant (depletion) or generation of electroactive species (production). The depletion or production process may then be monitored and may provide a direct measurement of the analyte concentration. In some cases, it may be desirable to provide a measurement of multiple analytes from a single biological sample. Further, multiple devices that are configured to measure a plurality of analytes may be efficiently made by separate manufacturing of component layers.

**SUMMARY OF THE INVENTION**

Embodiment transdermal sampling and analysis devices may include a base structure having at least one disruptor mounted on a base substrate, and a first electrode array. The at least one disruptor may be configured to generate a localized heat capable of altering permeability characteristics of a subject's skin. Embodiment transdermal sampling and analysis devices may also include a lid structure including a lid substrate having at least one hole configured to align with a portion of the at least one disruptor, and a second electrode array. In some embodiments, a region of the subject's skin is exposed to the portion of the at least one disruptor on contact with the lid substrate. Embodiment transdermal sampling and analysis devices may also include an adhesive structure including a core substrate coated with a thin continuous adhesive film on each external surface. The adhesive structure may have at least a first hole configured to direct interstitial fluid collected from the subject's skin to at least one of the first electrode array and the second electrode array. Embodiment transdermal sampling and analysis devices may also include at least one electrically conductive material passing through at least a second hole in the adhesive structure, and at least one conductive trace configured for external connection to one or more of a voltage/current source and a current detector. The at least one electrically conductive material may provide a conductive path between the base structure and the lid structure, and the at least one conductive trace may be configured for external connection to one or more of a voltage/current source and a current detector.

In some embodiments, the first electrode array may include at least one combined counter/reference electrode. In some embodiments, the second electrode array may include at least one working electrode. In some embodiments, the at least one working electrode may include a plurality of working electrodes each having a sensing layer configured to detect a distinct analyte. In some embodiments, the sensing layer of at least one of the plurality of working electrodes may include at least one cofactor. In some embodiments, at least one of the plurality of working electrodes may be coated with an anti-interferent barrier layer. In some embodiments, the anti-interferent barrier layer may be alginate. In some embodiments, each sensing layer may include an enzyme immobilized within a hydrogel, in which the enzyme causes a reaction to determine levels of a target analyte in the collected interstitial fluid.

In some embodiments, the plurality of working electrodes may include a first working electrode configured to detect glucose, and a second working electrode configured to detect an analyte selected from alcohol or lactate. In some embodiments, the plurality of working electrodes may include a first working electrode that is a glucose oxidase sensing layer, and a second working electrode that is an oxidoreductase sensing layer. In some embodiments, the oxidoreductase may be selected from alcohol dehydrogenases or lactate dehydrogenases.

In some embodiments, the base structure and the lid structure may be aligned such that the first electrode array is positioned directly opposite the second electrode array. In some embodiments, the at least one disruptor may have a serpentine configuration.

Embodiment methods of manufacturing a plurality of biosensing chips may include interference from a charged biological reducing species in a transdermal biosensor device may include forming a base wafer with a first polyimide film, a first electrically-conductive noble metal coating, and a first negative contrast photoresist layer, plating at least a first electrode on an exposed surface of the first electrically-conductive noble metal coating, forming a lid wafer with a second polyimide film, a second electrically-conductive noble metal coating, and a second negative contrast photoresist layer, fabricating at least a second electrode by selectively applying a sensing hydrogel to an exposed surface of the second electrically-conductive noble metal coating, forming a dual-sided adhesive (DSA) wafer comprising a polymer core film between two polymer release films, removing one of the two polymer release films such that the acrylic adhesive on a first surface of the polymer core film is exposed, attaching the lid wafer to the DSA wafer at the first surface of the polymer core film such that a DSA-lid stack is created, removing the other of the two polymer release films such that the acrylic adhesive on a second surface of the polymer core film is exposed, attaching the base wafer to the DSA-lid stack at the second surface of the polymer core film such that a combined structure is created, and laser cutting the combined structure to define individual biosensing chips. In some embodiments, the polymer core film may be coated with an acrylic adhesive on two surfaces.

Embodiment methods of manufacturing a plurality of biosensing chips may further include depositing a conductive material onto at least one area of the base wafer. In some embodiments, the deposited conductive material electrically connects the first and second electrically conductive noble metal coatings in the combined structure.

In some embodiments, fabricating at least a second electrode may include fabricating a plurality of working electrodes. In some embodiments, each sensing hydrogel of the plurality of working electrodes may include a distinct reagent configured to detect a target analyte within a biological fluid sample.

In some embodiments, the plurality of working electrodes may include at least a first working electrode configured to detect glucose, and at least a second working electrode configured to detect an analyte selected from alcohol or lactate. In some embodiments, the first electrically-conductive noble metal coating and the second electrically-conductive noble metal coating each include gold. In some embodiments, plating at least a first electrode on the exposed surface of the first electrically-conductive noble metal coating may include plating one or more region of pure silver, and electrochemically corroding a portion of the pure silver to make silver chloride.

In some embodiments, the first and second negative contrast photoresist layers may each include an epoxy-based photoresist material. In some embodiments, the first polyimide film may have a thickness of about 125 micrometers, and the second polyamide film may have a thickness of about 12-25 micrometers.

In some embodiments, generating the base wafer may include depositing a first polyimide film, the first electrically-conductive noble metal coating, and the first negative contrast photoresist layer on a first glass substrate. In some embodiments, generating the base wafer may include laminating a double-sided ultraviolet (UV) light release film onto the first glass substrate prior to depositing the first polyimide film, the first electrically-conductive noble metal coating, and the first negative contrast photoresist layer.

In some embodiments, generating the lid wafer may include depositing a second polyimide film, a second electrically-conductive noble metal coating, and a second negative contrast photoresist layer on a second glass substrate. In some embodiments, generating the base wafer may include laminating a double-sided ultraviolet (UV) light release film onto the second glass substrate prior to depositing the second polyimide film, the second electrically-conductive noble metal coating, and the second negative contrast photoresist layer.

## BRIEF DESCRIPTION OF THE DRAWINGS

The accompanying drawings, which are incorporated herein and constitute part of this specification, illustrate exemplary aspects of the invention, and together with the general description given above and the detailed description given below, serve to explain the features of the invention.

FIG. 1A is a component block diagram of a transdermal sampling and analysis device suitable for use with various disclosed embodiments.

FIG. 1B is a top view of another transdermal sampling and analysis device suitable for use with various disclosed embodiments.

FIG. 2 is a perspective view of a portion of a lid and base structure of another transdermal sampling and analysis device suitable for use with various disclosed embodiments.

FIG. 3 is a top view of a base structure and lid structure of an embodiment sampling and analysis device capable of dual analyte detection.

FIG. 4 is a vertical cross-section view of a portion of the transdermal sampling and analysis device shown in FIG. 3.

FIG. 5 is a top view of a base structure of another embodiment transdermal sampling and analysis device capable of dual analyte detection.

FIG. 6A is a vertical cross-section view of a portion of the transdermal sampling and analysis device shown in FIG. 5.

FIG. 6B is a top view of a base structure of another embodiment transdermal sampling and analysis device capable of detecting multiple analytes.

FIG. 7A is a vertical cross-sectional view of a portion of a schematic base wafer used to create embodiment three-layer biosensing chips.

FIG. 7B is a vertical cross-sectional view of a portion of a schematic dual-sided adhesive (DSA) wafer used to create embodiment three-layer biosensing chips.

FIG. 8 is a vertical cross-sectional view of a schematic lid wafer used to create embodiment three-layer biosensing chips.

FIG. 9 is a vertical cross-sectional view of a schematic DSA-lid stack made using the components shown in FIGs. 7B and 8.

FIG. 10A is a vertical cross-sectional view of a schematic combined structure used to create embodiment three-layer biosensing chips.

FIG. 10B is a vertical cross-sectional view of a schematic biosensing chip made using the components shown in FIG. 10A.

FIG. 11 is a process flow diagram illustrating an embodiment method of making a plurality of biosensing chips.

## DETAILED DESCRIPTION

The various embodiments will be described in detail with reference to the accompanying drawings. Wherever possible, the same reference numbers will be used throughout the drawings to refer to the same or like parts. References made to particular examples and implementations are for illustrative purposes, and are not intended to limit the scope of the disclosure or the claims. Alternate embodiments may be devised without departing from the scope of the disclosure. Additionally, well-known elements of the disclosure will not be described in detail or will be omitted so as not to obscure the relevant details of the disclosure.

The words "exemplary" and/or "example" are used herein to mean "serving as an example, instance, or illustration." Any embodiment described herein as "exemplary" and/or "example" is not necessarily to be construed as preferred or advantageous over other embodiments.

The term *"in situ"* is used herein to refer to tests and/or measurements performed using a natural composition/tissue in an environment that is at least partially subject to artificial control. For example, a novel transdermal biosensor may be said to be operating *in situ* when it is placed on the surface of a patient's skin while it disrupts the stratum corneum continuously to generate a biological fluid sample and analyze the generated biological fluid sample while the biosensor remains in place on the surface of the subject's skin.

Biosensors combine a biological sample with a physiochemical detector component (i.e., reagent) to allow for the detection of an analyte (that is, a substance or chemical constituent that is determined in an analytical procedure).

The signal generated as a result of interaction between the detector and the analyte may be electrical, optical, thermal, etc., is transformed by the means of suitable transducer element into a measurable parameter such as current or voltage. The biosensor selectivity against the target analyte may be determined by the physiochemical detector, while sensitivity may be dependent on the transducer.

In a number of biosensor applications, it may be beneficial to detect small quantities of multiple target analytes in a single, small volume using the same device or system. In this manner, efficiency may be maximized, and the patient need not be subjected to more than one extraction of biological fluid to perform such biosensing.

Compared to other detection methods, such as colorimetric methods, chemiluminescence, fluorimetric methods, high performance liquid chromatography (HPLC), and magnetic resonance spectroscopy, amperometric biosensors have shown advantages such as simplicity, portability, rapid response, high specificity, and simple operation. In particular, amperometric biosensors may be convenient for monitoring small molecule concentrations. Amperometric biosensors may be based on an enzyme that has the ability to catalyze a reaction involving the analyte. Such reactions may involve the consumption of an electroactive reactant and/or production of an electroactive product. The depletion of the electroactive reagent or production process of the electroactive product may be monitored amperometrically to provide a direct measurement of the analyte concentration. Specifically, electrons from the enzyme-catalyzed redox reaction may be transferred to an electrode (e.g., a working electrode), and the electric current flow resulting from the transfer of electrons may be used as a measure of the analyte. From the current flow level an analyte concentration in the original sample may be deduced. Amperometric biosensors measure the electric current generated between the working electrode and a counter electrode when the potential between them may be maintained at a constant level by means of a potentiostat. Such biosensors are typically based on enzymes that either consume oxygen, generate hydrogen peroxide, or indirectly produce a reduced form of a cofactor (e.g., NADH, NADPH, $FADH_2$, coenzymeQ, etc.) with the catalytic breakdown of a substrate.

Biosensors designed for patient (also referred to as a subject) home monitoring generally measure electric current produced by an enzyme-catalyzed redox reaction in an electrochemical cell involving an analyte of interest for medical purposes (e.g., glucose, etc.). The sample used for analysis may be a blood droplet collected from a subject's finger, arm, or other location on the body. In addition to being painful, these conventional biosensors are also designed in a manner so as to require a relatively large fluid sample to accurately determine analyte concentration. For example, the currently available conventional blood glucose biosensors require at least 300 nl of blood in order to analyze the blood glucose levels. To obtain these larger biological samples, painful and invasive procedures must be employed, which are not desirable.

Another disadvantage of the conventional biosensors is that they require several steps before they can analyze a biological sample. Conventional biosensors require loading a lancing device with a disposable tip, loading a test strip into an analyzer, breaching the skin, collecting the biological samples (e.g., blood), depositing the biological sample onto the test strip, and disposal of the sharp tip and blood-laden test strip. This multi-step process is time consuming and may cause contamination or loss of the biological sample during the collection and/or delivery.

The terms "transdermal sampling and analysis device" and "transdermal biosensor" are used interchangeably herein to refer to a one-step transdermal biosensor that provides *in situ* measurement of analyte concentrations from small quantities of the interstitial fluid collected from the capillary-like channels of a subject's skin. Examples of such transdermal sampling and analysis devices and methods for their manufacturer are described in: U.S. Patent No. 9,877,673, entitled "Transdermal Sampling and Analysis Device," in U.S. Patent Application No. 14/208,344, entitled "Methods of Manufacture to Optimize Performance of Transdermal Sampling and Analysis Device," in International Published Application No. WO2007/070093, entitled "Flexible Apparatus and Method for Monitoring and Delivery," in U.S. Patent No. 8,364,228, entitled "Apparatus and Method for Continuous Real-time Trace Biomolecular Sampling, Analysis, and Delivery," and in the publication entitled "Novel Non-Intrusive Trans-Dermal Remote Wireless Micro-Fluidic Monitoring System Applied to Continuous Glucose and Lactate Assays for Casualty and Combat Readiness Assessment" by John F. Currie, Michael M. Bodo and Frederick J. Pearce, RTO-MP-HFM-109:24-1, August 16, 2004, the contents of which are hereby incorporated by reference for the purpose of describing the device and methods disclosed therein.

The transdermal sampling and analysis devices/biosensors may also enable the entire process of analyzing a biological sample including disrupting the skin cells, collecting biological samples, reacting the biological sample with a biologically reactive element, and sensing the signals generated by the reaction in singular device. In contrast, in *in vitro* measurements, such as those performed using conventional blood tests or withdrawal techniques, a biological sample (e.g., ISF) may be collected, and subsequently tested for an analyte (or other measurable property) by submitting it to a remote assay or sensor. By incorporating a transdermal sampling device and analyzing device in a singular *in situ* test, a smaller biological sample may be utilized and the potential for contamination of the biological sample may be dramatically reduced. Moreover, the time required to obtain a sample and perform an analysis of the sample may be also reduced.

In such transdermal sampling and analysis devices/biosensors, a subject's stratum corneum may be disrupted through the application of localized heat to allow interstitial fluid to permeate from capillary-like channels in the subject's skin for sampling, collection, and analysis. The sampled and collected fluids may be tested for an analyte by reacting the collected fluids to a biologically reactive element, such as an enzyme (e.g., an oxidoreductase). The products of a biochemical reaction between the collected fluid sample and the biologically reactive element may be analyzed electrochemically to compute the concentration of the analyte (also referred to as "reactant") from either an electric potential or an electrical current. The amount of electric potential or current that is detected may be mapped to determine the concentration levels of analytes or characteristics of the collected fluid sample. Once the disruptor unit of the transdermal analysis and sensing devices/biosensors is removed from the skin, stratum corneum cells become impermeable again by returning to their original formation and closing the capillary-like channels.

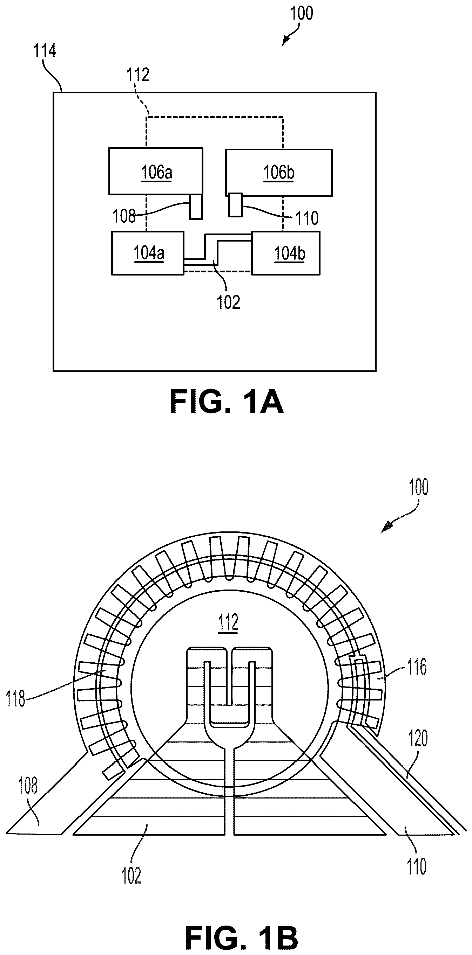

FIG. 1A is a block diagram illustrating the functional components of an example transdermal sampling and analysis device 100. A transdermal sampling and analysis device 100 may include a disruptor 102 connected to the positive and negative electrical poles of a signal generator 104a, 104b. In an embodiment, the disruptor 102 may function as a resistive element. The disruptor 102 produces heat as electrical current is applied through the disruptor 102. When placed on the skin, the localized heat generated by the disruptor 102 element may cause disruption to the skin cells facilitating the flow of interstitial fluid onto the surface of the transdermal sampling and analysis device 100. The disruptor 102 may be made from a variety of materials which exhibit the appropriate heating and control properties to provide the precise heating control properties required to disrupt the skin cells without damaging them. After a brief period of increased permeability due to the application of localized heat, the skin cells return to their normal function and may become impermeable again to retain their interstitial fluid.

The materials used to create the disruptor 102 may be selected for relative ease of manufacture as well as cost considerations. Materials such as titanium, tungsten, stainless steel, platinum and gold may be preferably used to form the disruptor 102. In a preferred embodiment, gold may be used to form the disruptor 102.

In an embodiment, when analyzing concentrations of an analyte in an obtained biological fluid sample (e.g., interstitial fluid), enzymatic reactions involving the analyte may yield electrons that may be captured to generate anodic current between a counter electrode 108 and a working electrode 110, also referred to as sensing electrodes 108, 110. Such enzymatic reactions may include, for example, the conversion of ethanol to acetaldehyde, of lactate to pyruvate, of glucose to gluconolactone, or others. The sensing electrodes 208, 210 may be coupled to electrically conductive paths 106a, 106b. The magnitude of the electrical current generated across the working electrode 110 and the counter electrode 108 as a result of this enzyme-catalyzed reaction may be proportional to the amount or concentration of the analyte contained in the obtained biological fluid sample. In an embodiment, a voltage potential may be applied to at least the working electrode 110 using a power generator (not shown). In some embodiments, the transdermal sampling and analysis device 100 may also include a reference electrode to provide a stable and well-known electrode potential. The high stability of the electrode potential may be reached, for example, by employing a redox system with constant (buffered or saturated) concentrations of each component of the redox reaction.

In such a scenario, the working electrode 110 may function as an anode and the counter electrode 108 may function as a cathode of an electrolytic cell, or vice versa. The magnitude of the current generated may be measured by an ammeter, the measurement of which may directly correlate to the amount (or concentration) of analyte in the collected biological fluid sample.

A transdermal sampling and analysis device 100 may further include a reservoir 112 for collecting and containing biological samples such as interstitial fluids that flow from capillary-like channels in disrupted stratum corneum. The reservoir 112 may be formed under the disruptor 102 and sensing electrodes 108, 110. When the transdermal sampling and analysis device 100 is place on the subject's skin with the disruptor 102 contacting the skin, the reservoir may effectively be positioned above the disruptor 102 and sensing electrodes 108, 110 to contain the released fluid sample. The reservoir 112 may include a cover or lid to more effectively contain the fluid sample. A reservoir 112 may be created using conventional methods known in the art, for example, by the buildup of material by additive process or by subtractive process such as photolithography. A substrate 114 may form the support upon which transdermal sampling and analysis device 100 components may be positioned or attached. Because the obtained biological fluid sample may be analyzed without removing the device from the subject, the process is referred to as an *in situ* process.

FIG. 1B illustrates an alternative embodiment of the transdermal sampling and analysis device 100. The transdermal sampling and analysis device 100 may include a disruptor 102 having a serpentine configuration, within a collection reservoir 112. Leads capable of coupling the disruptor 102 to a voltage/current source may be extended to the corners of the transdermal sampling and analysis device 100. The disruptor 102 may be also positioned within a hole in a lid layer so that the disruptor 102 may be exposed to and may directly contact the subject's skin for disruption of the stratum corneum and the production of a biological fluid sample. A sensing chamber 116 may form a circular shape around the periphery of the collection reservoir 112. The sensing chamber 116 may contain sensing channels 118 in a radial arrangement. The circular sensing channels 118 may guide the flow of a biological fluid sample through the circular-shaped sensing chamber 116. The sensing chamber 116 may provide the biological fluid sample over the entire surface of the counter electrode 108 and the working electrode 110. A reference electrode 120 may optionally be included. The disruptor 102, counter and working electrodes 108, 110, and optional reference electrode 120 may be all formed on a substrate layer.

With reference to FIGs. 1A and 1B, many different analysis techniques may be incorporated into the transdermal sampling and analysis unit 100 to determine the levels and concentrations of various analytes in a biological fluid sample. For example, amperometric, coulometric, potentiometric techniques may be each alternative techniques that may be incorporated into the transdermal sampling and analysis device 100 to determine levels or concentrations of analytes in a biological fluid sample. In addition, electrochemical impedance analysis techniques may be incorporated to detect the presence of particular antibodies in a biological fluid sample.

The counter and working electrodes 108, 110 may be made from any of a variety of materials which exhibit satisfactory conductivity characteristics and appropriate to the specific measurement used. In addition, the materials used to create the electrodes 108, 110, 120 may be selected for relative ease of manufacture as well as cost considerations. Examples of materials exhibiting satisfactory conductivity characteristics for use as the counter electrode 108 and the working electrode 110 may include gold, platinum, silver, carbon or other materials.

Selection of a substrate 114 for the transdermal sampling and analysis device 100 may depend on the coefficient of thermal expansion and conductivity of the material used to make the disruptor 102 of the transdermal sampling and analysis device 100. For example, the substrate 114 may be made of a material which has a coefficient of thermal expansion (CTE) that deviates from the CTE of the material used in the disruptor 102 by less than 50%, and preferably by less than 10%. In a further embodiment, the substrate 114 may be made of a material which has a coefficient of thermal conductivity (CTC) that is lower than 0.5 W/(m·K).

A sensing layer including an enzyme may be applied to the surface of at least one of the sensing electrodes 108, 110, such as the working electrode 110. By applying a voltage (or current) across the terminals of the disruptor 102, a precision-controlled heat may be produced and localized to the disruptor site. The localized heat may be applied against the subject's skin to alter the permeability of the skin cells and consequently create capillary-like channels. As the stratum corneum is disrupted, biological interstitial fluid may begin to flow through the stratum corneum into the reservoir 112 by capillary action of the structure. Interstitial fluid may flow out of the capillary-like channels into the reservoir 112 and over the surface of the counter electrode 108 and the working electrode 110. The obtained biological fluid sample (i.e., interstitial fluid) may come into contact with the sensing layer coating the surface of the working electrode 110, causing a chemical reaction that releases energy in the form of electrons. The electrons released due to the reaction may travel through the working electrode 110 towards the counter electrode 108, generating a current.

The transdermal sampling and analysis device 100 may be designed to deliver heat to the subject's skin with a power density of 1-10 W per $mm^2$. In a preferred embodiment the disruptor 102 delivers heat to the subject's skin with a power density of 2-5 W per $mm^2$. The transdermal sampling and analysis devices 100 may be made using a variety of different disruptor 102 configurations. The size and shape of the disruptor 102 may affect the resistive characteristics of the disruptor 102 and consequently, the ability of the disruptor 102 to generate a localized heat. In addition, the material selected to form the disruptor 102 may affect the resistive characteristics of the disruptor 102 and consequently, the ability of the disruptor 102 to generate a localized heat. As with sensing electrode 108, 110 material selection, disruptor 102 materials may be selected from a wide variety of materials exhibiting satisfactory electrical conductance/resistive properties such that sufficient heat may be generated when specific voltages are applied to the disruptor 102 leads. In addition, thermal conduction and resistance characteristics should be observed in an optimal disruptor 102 material. Finally, ease of manufacturing processing and cost may determine the final selection of disruptor material. For example, a disruptor 102 may be made of nichrome, titanium, tungsten, or gold. In a preferred embodiment, the disruptor 102 may be made from gold.

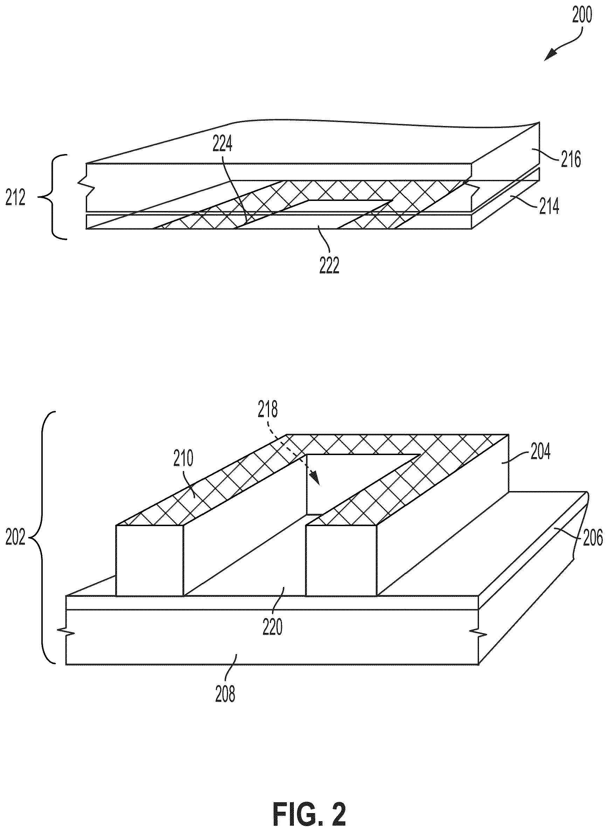

FIG. 2 illustrates a transdermal sampling and analysis device 200 according to an alternative embodiment. With reference to FIGs. 1A-2, the device 200 (not drawn to scale) may be formed using techniques similar to those discussed above with respect to transdermal sampling and analysis device 100. Representative components of the transdermal sampling and analysis device 200 may include a base structure 202 that includes a spacer layer of channel support structures 204 formed over a counter electrode 206, which are both formed over a base substrate 208. An adhesive (not shown) may be applied to the top surface 210 of the channel support structures 204. A lid structure 212 of the transdermal sampling and analysis device 200 may have a working electrode 214 patterned onto a lid substrate 216. A layer of analyte sensing reagent (not shown) may be applied to the surface of the working electrode 214.

The base structure 202 and lid structure 212 illustrate representative cross-section segments of a larger, three-dimensional device transdermal sampling and analysis device 200, and are not meant to limit the device 200 based on size or shape. Further, while the base structure 202 is shown with two spacer layer channel support structures 204, they are representative of any of a plurality of sets of channel support structures that may be formed across a larger base structure.

As the lid structure 212 is brought down into position over the channel support structures 204, the adhesive (not shown) on the channel support structures 204 may secure the lid structure 212 by contacting the working electrode 214. In this manner, channels 218 may be formed between exposed areas 220 of the counter electrode 206 and exposed areas 222 of the working electrode 214. Such exposed areas on both the working electrode 214 and the counter electrode 206 electrodes may be defined around the direct contact between the top surface 210 of the spacer layer channel support structures 204 and a corresponding contact area 224 on the surface of the working electrode 214.

In an alternative embodiment, an additional spacer layer may be applied atop the channel forming spacer layer (i.e., channel support structures). In the various embodiments, the second spacer layer may be recessed back from the channel support structures in order to provide lift space between the channel support structures and a substantial portion of the working electrode surface.

While not limited to particular dimensions, in some embodiments the second spacer layer may be approximately the same thickness as the channel forming spacer layer. In an embodiment, the total thickness of the channel forming spacer layer and second spacer layer may be approximately the same as that of the single channel forming spacer layer, such as that of channel support structures 204. In this manner, the depth of the sensing channels (i.e., vertical space between the counter and working electrodes) may remain the same to avoid requiring a greater amount of fluid to fill.

Amperometric biosensors utilize the electrical current produced when an oxidation or reduction reaction occurs at an electrode, such as one of the sensing electrodes 108, 110 (e.g., working electrode 110, 214, 309, 313); measurement of electrical current generated during the reaction is directly proportional to concentration of that species present in the particular sample. Thus, amperometric biosensors function by measuring the electrical current produced through the application of an electrical potential across working and reference electrodes that results from the electrocatalytic oxidation or reduction of the involved electroactive species. The magnitude of the measured electrical current is directly correlated to the concentration of a redox-active reagent or product in an enzymatic reaction. Generally, a biosensor for detection of L-lactate in tissue or blood focuses on enzymatic amperometric sensors owing to their simple design and performance.

The enzyme utilized in such biosensors may have the ability to catalyze a reaction involving the analyte with the consumption of an electroactive reactant and/or the production of an electroactive product. The depletion or production process is then monitored amperometrically and gives a direct measurement of the analyte concentration.

Examples of enzymes that may be used as the biologically reactive element to measure glucose are Glucose Oxidase (GOx) and glucose dehydrogenase. In an embodiment, GOx may be applied to the surface of a working electrode. The glucose molecules in the interstitial fluid may react with GOx covering the surfaces of the working electrode 210. Glucose oxidase catalyzes a breakdown of glucose in the interstitial fluid to gluconolactone, releasing electrons to a mediator such as $K_3Fe[CN]_6$.

The electron mediator may transfer electrons to the working electrode, where anodic potential has been applied such that the mediator may be oxidized. The oxidized mediator may be then able to accept another electron from the glucose conversion reaction to repeat the process. The electrons released in this oxidation reaction may travel through the working electrode towards a counter electrode, generating a current. The magnitude of the sensed electrical current generated by this reaction may be proportionally related to the concentration levels of glucose in the interstitial fluid. Thus, by determining the magnitude of current generated across the working and counter electrode, one may determine the relative amount of glucose in the obtained sample.

In order to work as a catalyst, certain enzymes require the presence of a redox cofactor. For example, an oxidized form of the redox cofactor (e.g., nicotinamide adenine dinucleotide ($NAD^+$), flavin adenine dinucleotide (FAD), etc.) may be the initial electron acceptor, and converted into a reduced form (e.g., NADH, $FADH_2$, etc.) during the reaction. In a typical reaction cycle using such an enzyme, a substrate ($X-H_2$) may bind to the enzyme active site and interact with the cofactor to generate an oxidized product (X) a, the reduced cofactor, and, in some reactions, $H^+$. This is shown in Eq. 1 below:

$$X\text{—}H_2 + \text{Enzyme—Cofactor } (ox.) \ \rightarrow \text{Enzyme—Cofactor } (red.) + X \ [+H^+] \quad (Eq. 1)$$

Some biosensors measure the amount of the reduced form of the cofactor that is generated during this reaction. In particular, $NAD^+$ is an important cofactor since $NAD^+$ participates in enzymatic catalysis of a large number of oxidoreductases, including more than 300 dehydrogenase enzymes. NAD-dependent oxidoreductases are widely used in bioprocesses and analytical applications. Examples of enzymes that require $NAD^+$ include, but are not limited to, alcohol dehydrogenase (ADH), lactate dehydrogenase (LDH), glyceraldehyde phosphate dehydrogenase, pyruvate dehydrogenase, $\alpha$-keto-glutamate dehydrogenase, isocitrate dehydrogenase, malate dehydrogenase, hydroxy-acyl-SCoA dehydrogenase, etc.

For example, biosensors based on alcohol dehydrogenase (ADH) may be of particular interest for ethanol analysis due to the toxicological and psychological effects of ethanol in the human body. ADH, which is selective for primary aliphatic and aromatic alcohols, catalyzes the conversion of ethanol to acetaldehyde by reduction of $NAD^+$. In this reaction, two hydrogen atoms and two electrons are removed from the ethanol molecule. One of the hydrogen atoms and both electrons are subsequently transferred to $NAD^+$, generating NADH and $H^+$.

As another example, lactate dehydrogenase (LDH) is an enzyme that may be of important medical significance for use in biosensors for lactate analysis. Specifically, an elevated lactate level may be an indication of lack of oxygen (hypoxia) or the presence of other conditions that cause excess production or insufficient clearing of lactate from the system. LDH has a high catalytic activity for conversion of lactate to pyruvate in the presence of a cofactor (NAD or NADP). In particular, in the presence of $NAD^+$, two hydrogen atoms and two electrons are removed from the lactate molecule. One of the hydrogen atoms and both electrons are transferred to $NAD^+$, generating NADH and $H^+$.

In a biosensor, for each of these example dehydrogenase reactions, NADH may transport electrons between the enzyme and a sensing electrode (e.g., working electrode 110, 214, 309, 313). At a sensing electrode surface, NADH is oxidized under the influence of an applied potential, regenerating $NAD^+$ and producing a current that is directly proportional to the concentration of analyte in the sample (e.g., ethanol or lactate).

The direct oxidation of NADH to regenerate $NAD^+$ at the sensing electrode takes place at considerable overpotentials. To enable oxidation at lower potentials, an electron mediator may be immobilized on the surface of the sensing electrode, such as within a polymer matrix. In some embodiments, the polymer matrix may be formed from mediator-coupled polymer chains (e.g., Fc-LPEI). The polymer matrix may be a hydrogel-that is, a polymeric network of interconnected hydrophilic polymer chains-created by cross-linking the enzyme with the mediator-coupled polymer chains. In this manner, the enzyme may be immobilized.

Other polymers that may be used to create a matrix that immobilizes the mediator and/or enzyme in the sensing layer include, but are not limited to, the following polyions: poly(styrene)-co-styrene sodium sulfonate (NaPSS); polyvinylsilane (PVS); poly{1-4[4-(3-carboxy-4-hydroxyphenylazo) benzenesulfonamido]-1,2-ethanediyl sodium salt} ; (PAZO); poly (1-acrylamido-1-methyl-l-propane sulfonic acid) (PAPSA); Poly[bis(4-phenyl)(2,4,6-trimethylphenyl) amine] (PTAA); poly(2-acrylamido-2-methyl-1-propanesulfonic acid) (PAMPSA); polystylenemethylenediethylmethylamine (PSMDEMA); poly(allylamine hydrochloride) (PAH); precursors to poly(p-phenylenevinylene) (Pre-PPVs); poly (diallyldimethylammonium chloride) (PDDA); polyetherimide (PEI); poly(p-pyridyl vinylene) (PHPyV); and sulfonated polyaniline (SPAn).

In such systems, the mediator may be reduced by the enzyme, with the reduced form of the mediator in turn being reoxidized at the surface of the sensing electrode surface to provide the amperometric signal. Examples of mediators that may be used in the biosensors of various embodiments may include transition metal compounds, conducting polymers, and organic dyes. Transition metal compound-based mediators may include complexes with ferrocene (i.e., "Fc") $(Fe(C_5H_5)_2)$, ferrocenecarboxaldehyde $(C_{11}H_{10}FeO)$, ferricyanide $(Fe(CN)_6^{3-})$, Prussian blue, cobalt phthalocyanine, ruthenium phthalocyanine, osmium complexes with a variety of redox polymers (e.g., osmium bipyridyl complex $(C_{20}H_{16}C_{12}N_4Os)$), or other transition metal compounds. Conducting polymer-based mediators may include poly(aniline)-poly(acrylate), poly(aniline)-poly(vinylsulfonate), poly(pyrrole), poly(pyrrole)-poly(vinylsulfonate), poly(vinylpyrrolidone), or other polymers. Organic dye-based mediators may include methylene green, Meldola blue, tetrathiafulvalene, thionine, tetracyanoquinodimethane (TCNQ), or quinine groups.

In various embodiments, the combination of the sensing electrode and a sensing layer disposed on the surface of the sensing electrode may form a solid-state mediated sensor, also referred to herein as a "sensing element." In various embodiments, the sensing layer may be formed by a polymer, a mediator conjugated (i.e., covalently bonded) to the polymer, and an enzyme immobilized by the polymer with conjugated mediator. In various embodiments, the sensing layer may be a hydrogel, created by cross-linking of the enzyme and the polymer with conjugated mediator.

In order to provide a more comprehensive set of information without substantially increasing the complexity of the device or the number or volume of biological sample, the transdermal analyte detection device according to various embodiments may be configured to detect multiple biomarker analytes within a single chamber.

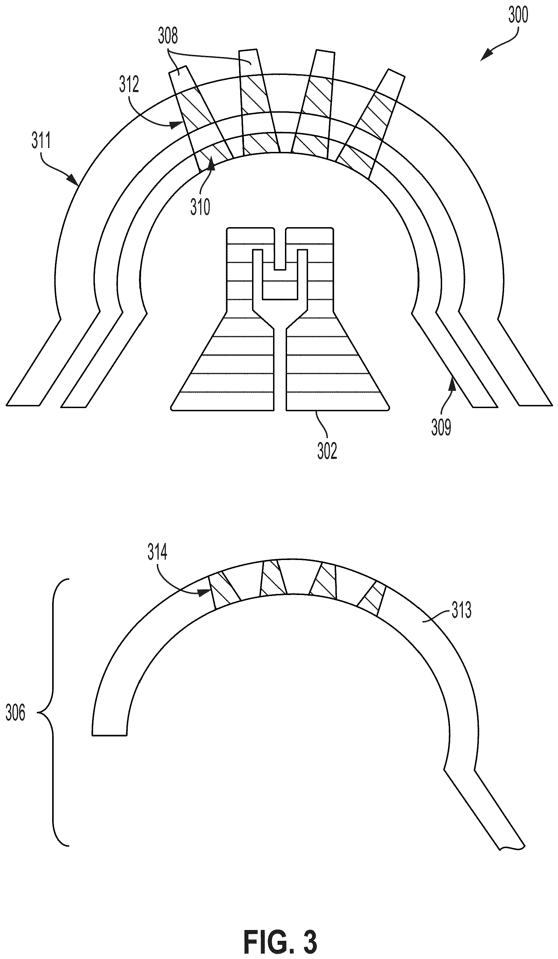

FIG. 3 illustrates a transdermal sampling and analysis device 300 capable of dual analyte detection according to various embodiments. With reference to FIGs. 1A-3, the device 300 may be formed using techniques similar to those discussed above with respect to transdermal sampling and analysis devices 100, 200. The transdermal sampling and analysis device 300 may include a disruptor 302, The disruptor 302 may have a serpentine configuration, similar to the disruptor 102. The disruptor 302 may be positioned within a collection reservoir, and within a hole in a lid layer so that the disruptor 302 may be exposed to and may directly contact the subject's skin for disruption of the stratum corneum and the production of a biological fluid sample. Leads capable of coupling the disruptor 302 to a voltage/current source may be extended to the corners of the transdermal sampling and analysis device 300.

The device may also include a base structure, a lid structure 306, and a sensing chamber. The sensing chamber, which may form a circular shape around the periphery of the collection reservoir, may be made up of well areas 308 into which the biological sample may be directed from the collection reservoir and contained in-between the base structure and the lid structure 306. The well areas 308 may be formed, for example, in-between one or more layer of channel support structures (e.g., 118).

The base structure may include a base substrate and a first working electrode 309 that, within the well areas 308, is coated with a compound to detect/sense a particular analyte in the sample, creating a first sensing electrode 310. The compound used for the first sensing electrode 310 may be referred to herein as a "first analyte sensing layer," and may include at least an enzyme. Examples of enzymes that may be used in the first sensing layer include glucose oxidase, glucose dehydrogenase, and other oxidoreductases, such as alcohol dehydrogenase, lactate dehydrogenase, etc. As described above, depending on the enzyme and desired reaction, the first sensing electrode 310 may include one or more cofactor, either within or layered on top of the first analyte sensing layer. While the particular cofactor may depend on the analyte of interest and the enzyme utilized in the first analyte sensing layer, examples include NAD$^+$, FAD, etc. Examples of the particular analyte that may be detected by the first sensing electrode 310 include, but are not limited to, glucose, lactate, alcohol, and others.

In various embodiments, the first sensing electrode 310 may occupy a portion of the surface of the base substrate within each well area 308. The base structure may also include an electrode 311 that serves as a counter/reference electrode 312 within the well areas 308 of the sensing chamber. The counter/reference electrode 312 may be capable of being electrically connected to a sensing electrode in order to act as a three-electrode system. Within each well area 308, the counter/reference electrode 312 may occupy part of the remainder of the base substrate that is not occupied by the first sensing electrode 310.

The lid structure 306 may include a lid substrate and a second working electrode 313 that, within the well areas 308, is coated with a compound to detect/sense a particular analyte in the sample, creating a second sensing electrode 314. The compound used for the second sensing electrode 314 may be referred to herein as a "second analyte sensing layer," and, similar to the first sensing layer, may include at least an enzyme (e.g., glucose oxidase, glucose dehydrogenase, alcohol dehydrogenase, lactate dehydrogenase, etc.).

As described above, depending on the enzyme and desired reaction, the second sensing electrode 310 may include one or more cofactor, either within or layered on top of the second analyte sensing layer. While the particular cofactor may depend on the analyte of interest and the enzyme utilized in the first analyte sensing layer, examples include $NAD^+$, FAD, etc.

In various embodiments, the particular analyte detected by the second sensing electrode 314 may be different from that of the first sensing electrode 310. For example, if the first sensing electrode 310 is configured to detect glucose, the second sensing electrode 314 may be configured to detect alcohol, lactate, or other relevant analyte.

In various embodiments, the second sensing electrode 314 may also be capable of being electrically connected to the counter/reference electrode 312, and may occupy the lid surface within the well area that is positioned directly above the counter/reference electrode 312.

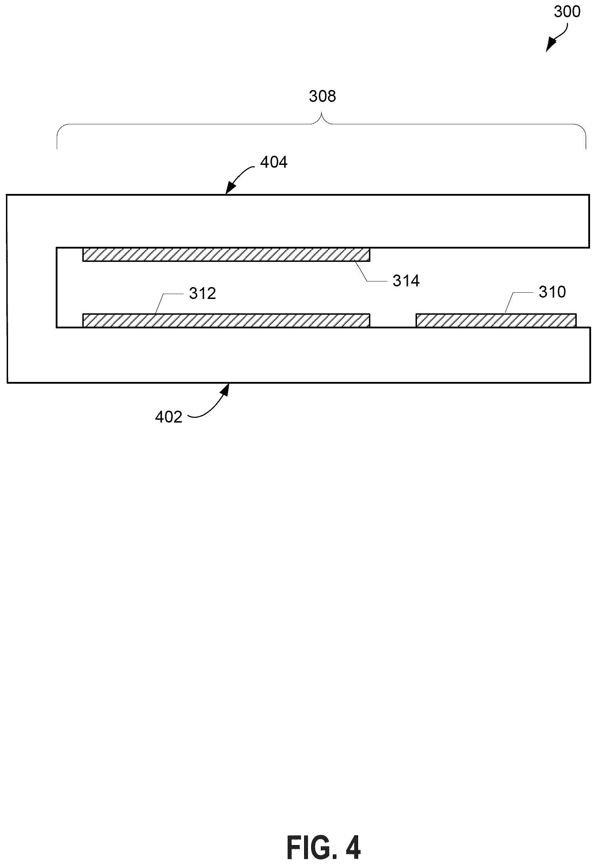

FIG. 4 illustrates a vertical cross-section of a well area 308 within the assembled transdermal sampling and analysis device 300 described in FIG. 3. With reference to FIGs. 1A-4, the transdermal analysis and sampling device 300 may include a base substrate 402, the first sensing electrode 310, the counter/reference electrode 312, a lid substrate 404, and the second sensing electrode 314. In various embodiments, the counter/reference electrode 312 may be positioned adjacent and co-planar to the first sensing electrode 310. The counter/reference electrode 312 may be disposed directly beneath the second sensing electrode 314 on an opposing plane. As described, the base structure of the transdermal sampling and analysis device 300 (e.g., the base substrate 402, the first sensing electrode 310, and the counter/reference electrode 312) may be separated from the lid structure (e.g., the lid substrate 404 and the second sensing electrode 314) by one or more layer of channel support structures (not shown). As illustrated in FIG. 4, the separation of the first and second sensing electrodes 310, 312 onto different layers (i.e., 402, 404) enables these electrodes to utilize different analyte sensing compounds.

In various embodiments, the base structure and the lid structure (e.g., 306) may be separately formed and joined during a final manufacturing step. In particular, such separation of the lid structure (e.g., 306) and base structure enables the first and second sensing electrodes 310, 314 to be individually coated with unique compounds (i.e., a first analyte sensing layer and a second analyte sensing layer) to detect/sense different analytes within the same biological sample. For example, when the biological sample is ISF, one sensing electrode (e.g., 310) may be configured to detect glucose, while the other sensing electrode (e.g., 314) may be configured to detect lactate or alcohol.

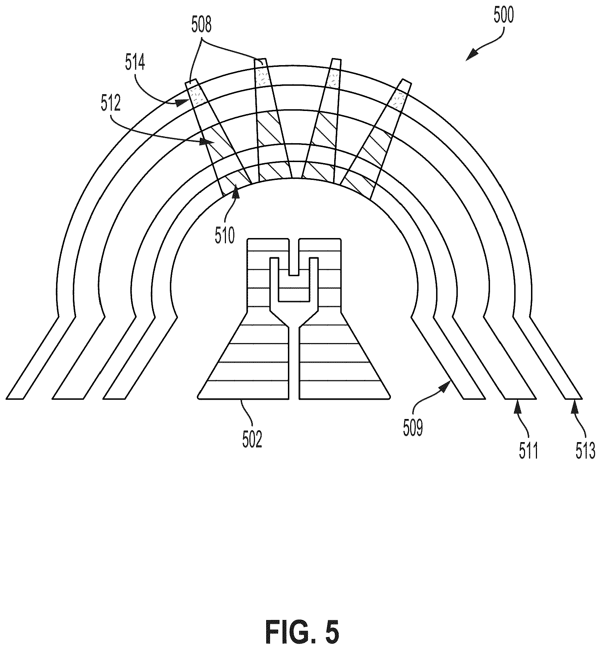

In other embodiments, the base structure may be formed to include two or more sensing electrodes (e.g., 310, 314) in addition to the counter/reference electrode (e.g., 312), all within the same plane. For example, the two or more sensing electrodes (e.g., 310, 314) and the counter/reference electrode (e.g., 312) may be formed together as part of the base structure or together as part of the lid structure (e.g., 306). FIG. 5 illustrates a transdermal sampling and analysis device 500 capable of dual analyte detection according to such alternative embodiments. With reference to FIGs. 1A-5, the device 500 may be formed using techniques similar to those discussed above with respect to transdermal sampling and analysis devices 100, 200, 300. The transdermal sampling and analysis device 500 may include a disruptor 502, which may have a serpentine configuration similar to those described above (e.g., 102, 302). The disruptor 502 may be positioned within a collection reservoir, and within a hole in a lid layer so that the disruptor 502 may be exposed to and may directly contact the subject's skin for disruption of the stratum corneum and the production of a biological fluid sample. Leads capable of coupling the disruptor 502 to a voltage/current source may be extended to the corners of the transdermal sampling and analysis device 500.

The transdermal sampling and analysis device 500 may also include a base structure, a lid structure (not shown), and a sensing chamber. The sensing chamber, which may form a circular shape around the periphery of the collection reservoir, may be made up of well areas 508 into which the biological sample is directed from the collection reservoir and contained in-between the base structure and the lid structure. In some embodiments, the well areas 508 may be formed in between one or more layer of channel support structures (e.g., 118).

The base structure may include a base substrate and a first working electrode 509 that, within the well areas 508, may be coated with a compound to detect/sense a particular analyte in the sample, creating a first sensing electrode 510. Examples of the particular analyte that may be detected by the first sensing electrode 510 include, but are not limited to, glucose, lactate, alcohol, etc. The compounds that may be used to create the first sensing electrode 510 include, but are not limited to glucose oxidase, glucose dehydrogenase, alcohol dehydrogenase, lactate dehydrogenase, and others.

In various embodiments, the first sensing electrode 510 may occupy a portion of the surface of the base substrate within each well area 508.

The base structure may also include an electrode 511 that serves as a counter/reference electrode 512 within the well areas 508. The counter/reference electrode 512 may be capable of being electrically connected to a sensing electrode in order to act as a three-electrode system. Within each well area 508, the counter/reference electrode 512 may occupy part of the remainder of the base substrate that is not occupied by the first sensing electrode 510.

The base structure may also include a second working electrode 513 that, within the well areas 508, may be coated with a compound to detect/sense a particular analyte in the sample, creating a second sensing electrode 514. In various embodiments, the particular analyte detected by the second sensing electrodes 514 may be different from that of the first sensing electrode 510. For example, if the first sensing electrode 510 is configured to detect glucose, the second sensing electrode 514 may be configured to detect alcohol, lactate, or other analyte.

The second sensing electrode 514 may also be capable of being electrically connected to the counter/reference electrode 512. Within each well area 508, the second sensing electrode 514 may occupy part of the remainder of the base substrate that is not occupied by the first sensing electrode 510 and the counter/reference electrode 512.

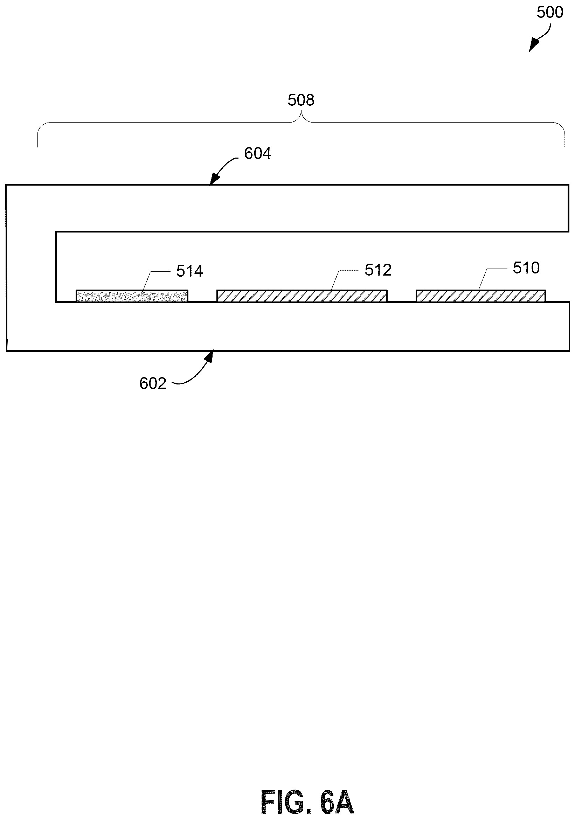

FIG. 6A illustrates a vertical cross-section of a well area 508 within the assembled transdermal sampling and analysis device 500 described in FIG. 5. With reference to FIGs. 1A-6A, the transdermal analysis and sampling device 500 may include a base substrate 602, the first sensing electrode 510, the counter/reference electrode 512, the second sensing electrode 514, and a lid substrate 604. In various embodiments, the counter/reference electrode 512 may be positioned adjacent and co-planar to both the first sensing electrode 510 and the second sensing electrode 514. As described, the base structure of the transdermal sampling and analysis device 500 (e.g., the base substrate 602, the first and second sensing electrodes 510, 514, and the counter/reference electrode 512) may be separated from the lid structure (e.g., the lid substrate 604 by one or more layer of channel support structures (not shown). One of ordinary skill in the art would recognize that while FIG. 6A illustrates the counter/reference electrode 512 positioned adjacent and co-planar to both the first sensing electrode 510 and the second sensing electrode 514 on the base structure 602, in other embodiments the counter/reference electrode 512 positioned adjacent and co-planar to both the first sensing electrode 510 and the second sensing electrode 514 on the lid structure 604.

Operation of the biosensor devices 300, 500 may involve extracting a biological sample from the subject via the disruptor (e.g., 302, 502), which may be directed to and contained within the plurality of well areas (e.g., 308, 508) in-between and giving access to the base and lid surfaces. Once a well fills with the biological sample, the sensing electrodes may be sequentially activated while being connected to a counter/reference electrode for rapid detection of the respective analytes. For example, the first sensing electrode (e.g., 310, 510) may be activated, enabling measurement of a first analyte in the sample while connected to the counter/reference electrode (e.g., 312, 512) Once the measurement of the first analyte is complete, which may take up to 10 seconds, an electrical switch may activate the second sensing electrode (e.g., 314, 514) to measure a second analyte in the sample while connected to the counter/reference electrode (e.g., 312, 512).

While described herein with respect to two sensing electrodes capable of detecting two different analytes, such disclosure is not meant to limit the embodiment devices to a particular number. That is, the various embodiment transdermal sampling and analysis devices may be made to include more than two sensing electrodes, and capable of detecting more than two different analytes.

Since different subjects may have different skin thickness levels, calibration of the transdermal sampling and analysis device 100, 200, 300, 500 may be required to generate sufficient heat to obtain the optimal amounts of biological fluid samples with the least amount of sensation. Thus, the level and duration of the temperature of the disruptor (e.g., 102, 302, 502), may be adjusted for different subjects. Preferably, disruption of the skin may occur when heat of 140°C from the disruptor (e.g., 102, 302, 502) may be supplied to the skin surface for duration of about 140 ms.

Due to the integration of collection and analysis in the same unit, various biological species in the ISF may interfere with the analyte measurements in the transdermal biosensor device of various embodiments. That is, in a system in which the mediator or enzyme is intended to be reduced by the oxidation of the cofactor, may instead be reduced by redox species in ISF that may be strong reducing agents. This transfer of electrons by other molecules may cause the amount of charge measured from the electrodes to no longer be in proportion with the levels of analyte. Therefore, in some embodiments, the sensing element may also include at least one anti-interferent barrier layer applied over the first and/or second analyte sensing layer. The barrier layer may prevent or limit interfering redox species in the ISF from reaching the corresponding sensing electrode.

In some embodiments, the barrier layer may be charged, and charge type repulsion with charged biological reducing species (i.e., interfering species) prevents interaction between the reducing species and the mediator in the redox hydrogel. The charged barrier layer may be associated with the analyte sensing layer using a variety of forces, such as charge interaction, covalent bonding, van der Waals forces, etc. In various embodiments, the at least one barrier layer may be formed using a variety of materials, including but not limited to alginates, or other anionic naturally occurring polymers. Such materials may be applied to the analyte sensing layer using processes that include, but are not limited to, chemical and physical deposition processes.

Parameters for creating the first and second sensing electrodes using at least the first and second analyte sensing layers according to the various embodiments, including concentration, temperature, and time, may be adjusted to achieve optimal results.

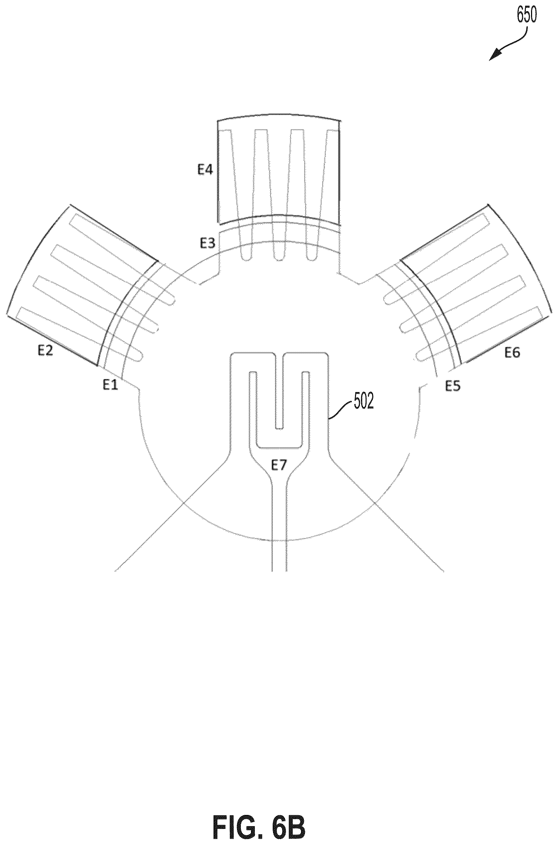

FIG. 6B illustrates a transdermal sampling and analysis device 650 capable of detecting a plurality of analytes according to alternative embodiments. With reference to FIGs. 1A-6B, the device 650 may be formed using techniques similar to those discussed above with respect to transdermal sampling and analysis devices 100, 200, 300, 500. The transdermal sampling and analysis device 650 may include a disruptor 502, which may have a serpentine configuration similar to those described above (e.g., 102, 302). The disruptor 502 may be positioned within a collection reservoir, and within a hole in a lid layer so that the disruptor 502 may be exposed to and may directly contact the subject's skin for disruption of the stratum corneum and the production of a biological fluid sample. Leads capable of coupling the disruptor 502 to a voltage/current source may be extended to the corners of the transdermal sampling and analysis device 650.

The transdermal sampling and analysis device 650 may also include a base structure, a lid structure (not shown), and a sensing chamber. The sensing chamber, which may form a circular shape around the periphery of the collection reservoir, may be made up of well areas into which the biological sample is directed from the collection reservoir and contained in-between the base structure and the lid structure.

The base structure may include a base substrate and a plurality of electrodes E1-E7 that may each be coated with a different compound to create a specific working electrode or a counter/reference electrode. In some embodiments, a portion of the electrodes E1-E7 may be coated with the same compound as each other, while in other embodiments each electrode E1-E7 may be coated with a distinct compound.

The compounds that may be used in coatings to create one or more sensing electrode among the electrodes E1-E7 include, but are not limited to glucose oxidase, glucose dehydrogenase, alcohol dehydrogenase, lactate dehydrogenase, and others.

Further, in some embodiments, up to two more pairs of electrodes may be added along the circumference of the collection reservoir, for a total of eleven (11) electrodes. In some embodiments, one of the electrodes (E7) may be positioned in a region adjacent the disruptor 502.

In various alternative embodiments, transdermal sampling and analysis devices may be constructed using at least one three-layer biosensing chip. In particular, three wafers may be separately created, each corresponding to a layer of the chip, and assembled to create multiple sealed electrochemical chips. Such electrochemical chips may be incorporated into devices that perform the transdermal sampling and analysis functions described above.

The first layer may be a base structure that includes at least one disruptor mounted on a first substrate, at least one electrode configured concentrically around the disruptor, and conductive traces to allow external connections to electrical current and voltage sources and/or detectors. In some embodiments, the at least one electrode of the first layer may have working, counter, reference, and/or sensing functions.

The second layer according to various embodiments may be a lid structure, and may contain at least one hole of a size comparable to at least a portion of the disruptor, and through which a small portion a subject's skin is exposed on contact, at least one electrode configured concentrically around the hole, and conductive traces to allow external connections to electrical current and voltage sources and/or detectors. In some embodiments, the at least one electrode of the second layer may have working, counter, reference, and/or sensing functions, and may be the same as or different from the at least one electrode of the second layer.

The third layer according to various embodiments may be an adhesive structure, and may include a substrate with thin continuous adhesive films on both sides of the substrate. The third layer may also include multiple holes for electrically conducting material providing conductive paths between the first layer and the second layer. Further, the third layer may also include multiple holes for directing the flow of interstitial fluid collected from the skin and to the electrical current and voltage sources and/or detectors.

In various embodiments, the three-layer biosensing chip may include one or multiple electrochemical cells, integrating biosensing/analysis of collected interstitial fluid within a diameter of around 1 mm and a depth of around 0.05 mm. A transdermal sampling and analysis device containing at least one three-layer biosensing chip may allow leakproof contact with the stratum corneum layer of the skin. The device may passively draw interstitial fluid based on the push small hydrostatic pressure beneath the stratum corneum, and the pull of surface tension of the internal sensor surfaces. Within the device, the interstitial fluid may be passively guided with capillary hydro fluidics towards enclosed channels containing multiple electrodes, irrespective of the sensor orientation in the gravitation field. The biosensing chip may also ensure that all electrochemical cells are covered with interstitial fluid, without turbulence or flow during the entirety of the analysis.

The various embodiments may include manufacturing separate adhesive, lid, and base wafers, which are assembled into a final structure and cut to create a plurality of biosensing chips for use in transdermal sampling and analysis devices.

To create a base wafer according to various embodiments, a positive contrast, two-sided ultraviolet (UV) light release film may be laminated onto a clean glass substrate. The UV light release film may be, for example, a double-sided UV tape. A thin polyimide film (e.g., Kaneka Apical®) may then be laminated on top of the UV light release film. In various embodiments, the polyimide film used in the base wafer may be around 125 $\mu$m thick, although thicker or thinner films may be used.

An electrically-conductive thin noble metal film (e.g., gold) may be evaporated onto the polyimide film under high vacuum, and a positive contrast photoresist may be spun on top of the metal. While the noble metal coating may be referred to in the various embodiments as gold, other noble metals that may be used include rhenium, ruthenium, rhodium, palladium, silver, osmium, iridium, and/or platinum.

The positive contrast photoresist may be, for example, polymethylmethacrylate (PMMA). The wafer may be baked to remove any solvent, and placed in a mask aligner. In various embodiments, a photomask may be aligned to the wafer, and the assembly may be exposed to UV light. In locations in which the photomask is transparent, the photoresist is directly exposed to the UV light, making the photomask soluble in developer solution.

The growing base wafer assembly may be removed from the mask aligner, and spun under three chemical sprays. Specifically, the first chemical spray may be a developer solution that removes the exposed photoresist, revealing the gold film underneath. The second chemical spray may etch away the exposed gold, leaving the precise pattern of the photomask as still containing gold film covered by the photoresist. The third chemical spray may dissolve all remaining photoresist, leaving the gold film in the pattern of the photomask. In various embodiments, such pattern may include areas defined for later electrode formation. The wafer may then be rinsed in deionized water, and dried (e.g., using a dry nitrogen jet).

In various embodiments, a negative contrast photoresist may be spun onto the wafer. In some embodiments, the negative contrast photoresist may be an epoxy-based material (e.g., KMPR®).

The wafer assembly may be baked to remove any solvent, and placed in a mask aligner. In the mask aligner a second photomask with a new pattern may be aligned to the first pattern, and exposed to UV light. In locations in which the photomask is transparent, the negative photoresist is directly exposed to the UV light, making it insoluble in developer solution. The wafer may be removed from the mask aligner, and sprayed with developer solution on a spinner to remove the unexposed negative photoresist. The wafer assembly may then be rinsed with deionized water, and baked dry.

A positive photoresist may be spun onto the wafer assembly. The wafer assembly may be baked to remove remaining solvent, and placed back in a mask aligner. Using a third photomask precisely aligned to the previous two patterns, the wafer may be exposed to UV light. The third photomask may be transparent in areas where gold surfaces are to be electroplated. That is, in such areas, the photoresist may be directly exposed to the UV light and rendered soluble in developer solution.

In various embodiments, the wafer may be returned to the spinner, spun under a developer solution that removes the exposed positive photoresist, and rinsed with deionized water. In some embodiments, the wafer may be subjected to a gas plasma reaction chamber where the assembly is bombarded with ions to automatically clean the exposed gold surfaces.

The wafer assembly may be transferred to one or more electrochemical baths to create the selected number of electrodes per cell. In various embodiments, different metals may be used for the electrodes, including gold, platinum, silver, graphite, and/or rhodium. In some embodiments, the metal(s) may be deposited separately or built-up by plating. For example, at least one counter/reference electrode may be formed by plating pure silver d to a desired thickness on the exposed gold surface, followed by a controlled electro-corrosion of half of the silver layer to form silver chloride.

The electrodes formed on the base wafer may be coated with various layers (e.g., a sensing layer(s), anti-interferent barrier(s), etc.) using techniques including precision droplet casting, spray coating, or layer-by-layer film assembly, etc.

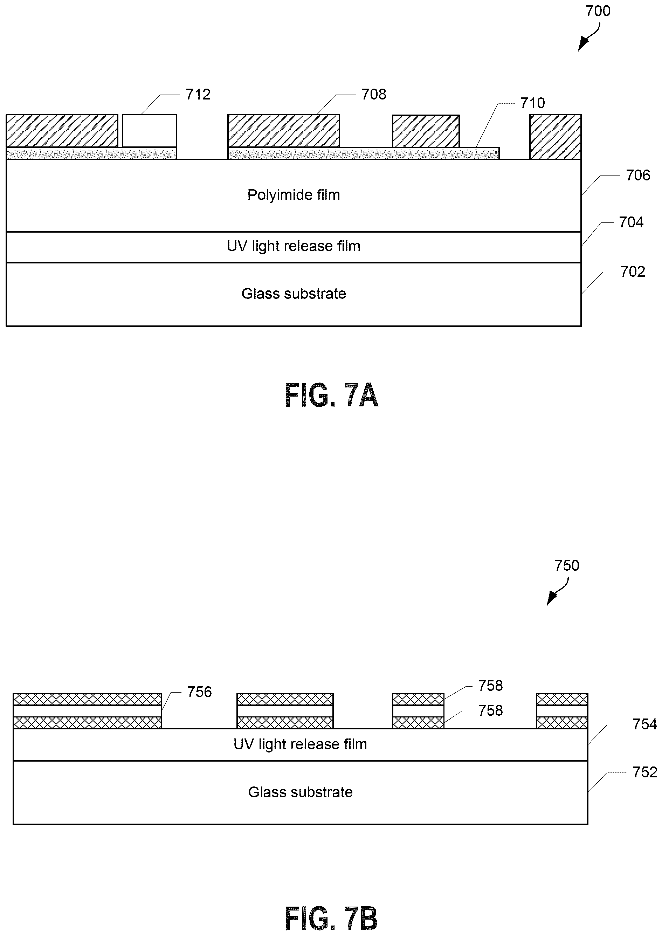

FIG. 7A illustrates a vertical cross-sectional view of a schematic base wafer 700 made in the process of manufacturing a plurality of three-layer biosensing chips. With reference to FIGs. 1A-7A, the constructed base wafer 700 may include a glass substrate 702, a UV light release film 704, and a polyimide film 706. The base wafer 700 may further include patterned regions of epoxy-based photoresist 708 (e.g., KMPR®) and gold coating 710. The base wafer 700 may further include at least one combined counter/reference electrode 712.

An adhesive wafer according to various embodiments may be, for example, a dual-sided adhesive (DSA). To create a DSA wafer according to some embodiments, a UV release film may be bonded onto a glass substrate, then covered by a DSA film. In various embodiments, the DSA film is a sandwich structure made up of two thin polymer release films and a thin polymer core film coated on each surface with an acrylic adhesive. Some or all of the thin polymer release films and the polymer core film may be made, for example, of polyethylene terephthalate (PET).

In various embodiments, a scanning laser beam may be used to make a pattern of precise holes through the DSA film that will align with features on both the base and the lid wafers when assembled. In particular, such holes may provide conductive paths between the lid and the base layers, to an external voltage/current source and/or current detector, and for directing the flow of biological fluid (e.g., interstitial fluid) to multiple electrode arrays.

FIG. 7B illustrates a vertical cross-sectional view of a schematic DSA wafer 750 made in the manufacturing process for a plurality of three-layer biosensing chips. With reference to FIGs. 1A-7B, the DSA wafer 750 may include a glass substrate 752, a UV light release film 754, and patterned regions of DSA film. The DSA film may be made of a thin polymer core film 756 that is coated on each external surface with an acrylic adhesive, and sandwiched between two thin polymer release films 758.

To create a lid wafer according to various embodiments, a positive contrast two-sided UV light release film (e.g., double sided UV tape) may be laminated onto a clean glass substrate. A thin polyimide film (e.g., Keneka Apical®) may be laminated on top of the UV light release film. In various embodiments, the polyimide film used to create the lid wafer may be thinner than that of the base wafer. In particular, the polyimide film may have a thickness of around 12-25 micrometers, although thicker or thinner films may be used.

An electrically-conducting thin gold coating may be evaporated onto the polyimide film under high vacuum, and a positive contrast photoresist may be spun on top of the gold coating. The positive contrast photoresist may be, for example, polymethylmethacrylate (PMMA). The wafer may be baked to remove any solvent, and placed in a mask aligner.

In various embodiments, a photomask may be precisely aligned to the wafer, and the assembly may be exposed to UV light. In locations in which the photomask is transparent, the photoresist is directly exposed to the UV light, making it soluble in developer solution.

The wafer assembly may be removed from the mask aligner, and spun under three chemical sprays. The first chemical spray may be a developer solution that removes the exposed photoresist, revealing the gold coating underneath. The second chemical spray may etch away the exposed gold, leaving the precise pattern of the photomask as still containing gold coating covered by the photoresist.

The third chemical spray may dissolve all remaining photoresist, leaving the gold coating in the pattern of the photomask. In various embodiments, such pattern may include areas defined for later electrode formation. The wafer may then be rinsed in deionized water, and dried (e.g., using a dry nitrogen jet).

In various embodiments, a negative contrast photoresist may be spun onto the wafer assembly. In some embodiments, the negative contrast photoresist may be an epoxy-based material (e.g., KMPR®).

The wafer may be baked to remove any solvent, and placed in a mask aligner. In the mask aligner a second photomask with a new pattern may be aligned precisely to the first pattern, and exposed to UV light. In locations in which the photomask is transparent, the negative photoresist is directly exposed to the UV light, making it insoluble in developer solution. In particular, the negative photoresist may be patterned to define an area(s) for one or more electrochemical working electrode(s).

The wafer assembly may be removed from the mask aligner, and on a spinner, sprayed with developer solution to remove the unexposed negative photoresist. The wafer may then be rinsed with deionized water, and baked dry. In a spinner, the wafer may be subjected to a chemical spray that dissolves all remaining positive photoresist, and then rinsed in deionized water and dried.

A scanning laser beam may cut through the polyimide layer to the middle of the UV light release tape. In various embodiments, the wafer assembly may then be subjected to a gas plasma reaction chamber, where it is bombarded with ions to automatically clean the exposed gold surfaces. One or multiple electrodes may be formed on the lid wafer by selectively coating one or more area of the exposed gold surface. Such coating may be done using techniques such precision droplet casting, spray coating, or layer-by-layer assembly.

In particular, one or more coating material each containing one or more reagent (e.g., a compound to detect/sense a particular analyte in a sample) may be applied by computer-controlled drop dispensing onto the cleaned exposed gold surface(s). The droplet may dry quickly leaving a uniform solid membrane coating, thereby creating working electrode(s). In some embodiments, such coating may be a polymer matrix, such as a hydrogel, that loosely holds enzymes and their cofactors onto an electrode surface. For example, the coating for one working electrode may be a hydrogel of LPEI that contains glucose dehydrogenase as a reagent, and the cofactor $NAD^+$, while the coating for another working electrode may be a hydrogel of LPEI that contains alcohol dehydrogenase as a reagent, and the cofactor $NAD^+$. Examples of the particular analyte that may be detected by the electrode(s) fabricated on the lid wafer may include, but are not limited to, glucose, lactate, alcohol, and others. In some embodiments, additional coatings may be applied to the surfaces of the working electrode(s), such as an anti-interferent barrier layer (e.g., alginate).

In various embodiments, the electrode configuration and the number of analytes that may be measured, is limited only by the number and position of working electrode(s) within each chip. Further, in various embodiments, the number of working electrode(s) formed on the lid wafer for each chip may be the same as or different from the number of counter/reference electrodes formed on the corresponding base wafer for each chip.

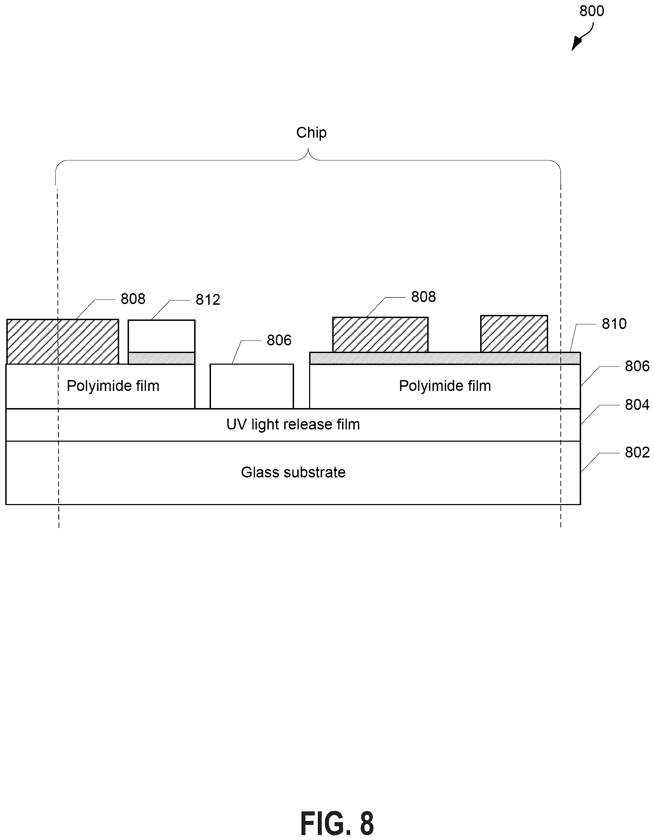

FIG. 8 illustrates a vertical cross-sectional view of a schematic lid wafer 800 created in the manufacture of a plurality of three-layer biosensing chips according to an embodiment. With reference to FIGs. 1A-8, the constructed lid wafer 800 may include a glass substrate 802, a UV light release film 804, and cut areas of thin polyimide film 806. The lid wafer 800 may also include patterned regions of epoxy-based photoresist 808 (e.g., KMPR®) and gold layer 810. The lid wafer 800 may further include at least one working electrode 812, which may be made of one or more layers (e.g., hydrogel) on a section of the gold layer 810.

In various embodiments, a base wafer, a DSA wafer, and a lid wafer as described above may be combined into a single structure used to make the plurality of biosensing chips.

The DSA wafer may be exposed to UV light through an aligned photomask, which causes the adhesion of the UV release film to decrease in areas exposed to the UV light (e.g., where the photomask is transparent). The patterned DSA wafer may be peeled off and placed on a clean glass substrate.

The top thin polymer release film of the new DSA wafer may be removed, exposing the polymer core film coated with the acrylic adhesive.

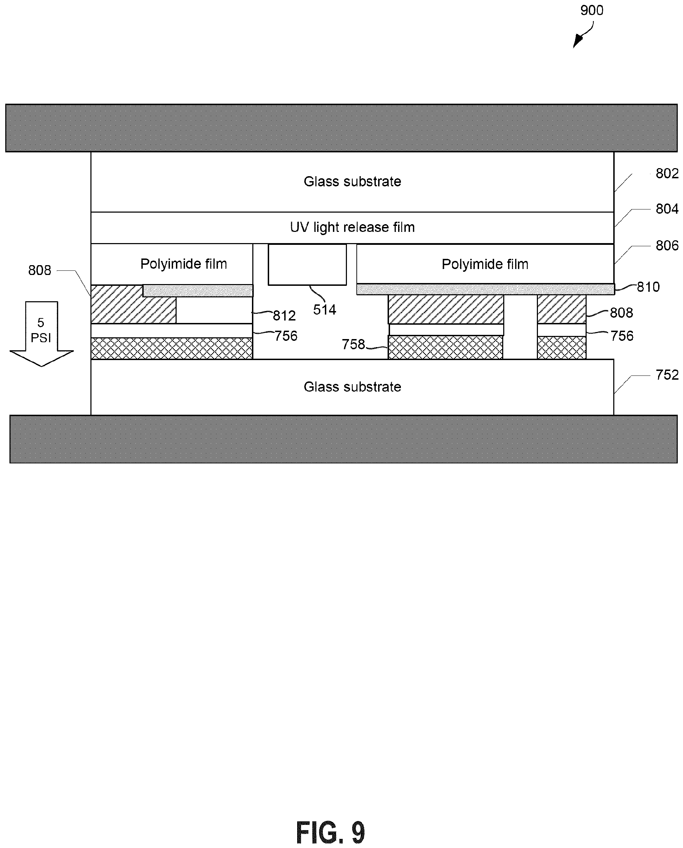

The DSA wafer and the lid wafer may then be pressed together at around 5 psi to create a combined DSA-lid stack 900, as illustrated in the schematic vertical cross sectional view of FIG. 9. With reference to FIGs. 1A-9, the DSA-lid stack 900 includes components of the lid wafer (e.g., 800) and the DSA wafer (e.g., 750). Specifically, the DSA-lid stack 900 may include the glass substrate 802, the UV light release film 804, and cut areas of polyimide film 806. The lid wafer 800 may also include patterned regions of epoxy-based photoresist 808 (e.g., KMPR®), gold layer 810, and at least one working electrode 812 (e.g., one or more layer deposited on gold surface). The DSA-lid stack may further include the glass wafer 752, and the patterned regions of DSA film. Specifically, such DSA film may include the thin polymer core film 756 coated with an acrylic adhesive, and one remaining thin polymer release film 758. The lid wafer and the DSA wafer may be pressed together, for example, using around 5 psi.

In various embodiments, the bottom glass substrate that was originally part of the DSA wafer may be removed from the DSA-lid stack.

A combined structure may then be generated in various embodiments using the DSA-lid stack and the base wafer. Taking the base wafer, droplets of a precise volume silver paste may be applied at specific points. To prepare the DSA-lid stack for final assembly, the remaining top thin polymer release film may be removed, exposing the polymer core film coated with the acrylic adhesive.

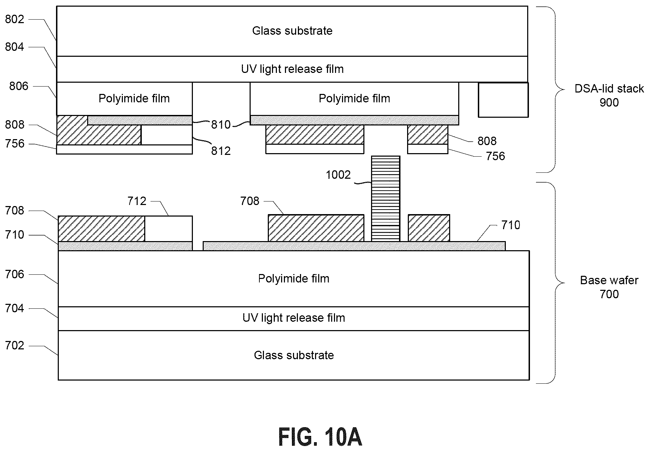

The position of the base wafer components and the DSA-lid stack components prior to final assembly are illustrated in the schematic vertical cross sectional view of FIG. 10A according to various embodiments. With reference to FIGs. 1A-10A, the components of the base wafer (e.g., 700) may include the glass substrate 702, the UV light release film 704, the polyimide film 706, patterned regions of the epoxy-based photoresist 708 (e.g., KMPR®), the gold layer 710, and at least one combined counter/reference electrode 712.

The components of the DSA-lid stack (e.g., 900) may include the glass substrate 802, the UV light release film 804, cut areas of polyimide film 806, patterned regions of epoxy-based photoresist 808 (e.g., KMPR®), gold coating 810, at least one working electrode 812 (e.g., one or more layer deposited on gold surface), and patterned regions of the DSA film. The DSA film may include the thin polymer core film 756 coated with an acrylic adhesive.

Silver paste 1002 deposited on the base wafer 700 may be positioned between a gold layer of the base (e.g., 710) and a gold layer of the DSA-lid structure (e.g., 810).

In various embodiments, the base wafer and the DSA-lid stack may aligned and pressed together to create a final (i.e., combined) structure. Specifically, once assembled, the silver paste deposited onto the base wafer may electrically connect the top and bottom gold conductive layers (i.e., on the base wafer and the DSA-lid stack). In some embodiments, the combined structure may be subjected to pressure (e.g., 30 psi) to ensure uniform adhesion of the complete device. The UV light release film on the base wafer may be exposed to UV light through the glass substrate underneath. In this manner, the polyimide film of the base wafer may be detached from the glass substrate.

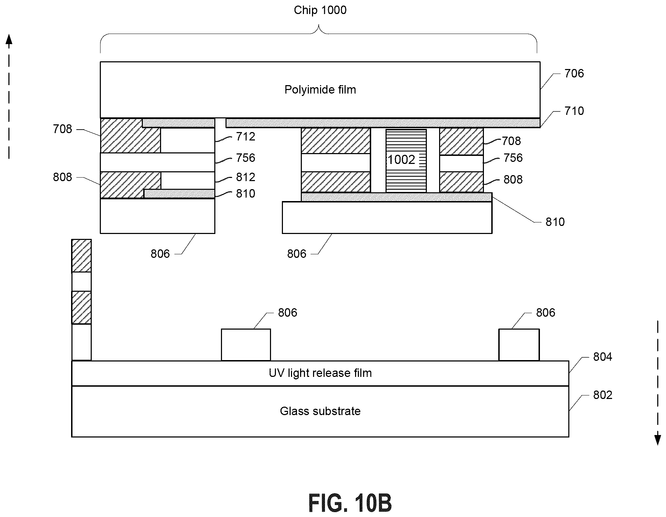

A laser may be used to cut completely through the two polyimide films and the layers within to define the separate biosensing chips. Finally, the UV light release film of the DSA-lid stack may be exposed to UV light by illuminating through the glass substrate, enabling the polyamide film of the DSA-lid stack structure to be detached from the glass substrate. In this manner, components that are not part of the chip functioning (e.g., the glass substrate, the UV light release layer, sections of the polyimide film, etc.) may be removed, as shown in FIG. 10B. With reference to FIGs. 1A-10B, a resulting biosensing chip 1000 may include the polyimide film 706, and patterned regions of the epoxy-based photoresist 708 and gold layer 710 that were part of the base wafer 700.

The chip 1000 may also include, from the DSA-lid stack 900, the working electrode(s) 812, and patterned regions of the thin polymer core film 756, epoxy-based photoresist 808 (e.g., KMPR®), gold layer 810, and polyamide film 806. The chip 1000 may further include a deposited conductive material 1002 (e.g., silver) that connects the gold layers 710 and 810. In various embodiments, removed components include remaining regions of the polyamide film 806, the UV light release film 804, and the glass substrate 802.

Using the techniques described herein, a plurality of biosensing chips may be created at once, removed individually, and incorporated into cartridges to make transdermal sampling and analysis devices.

In various embodiments, possible polymers which can serve as substrate material, insulating material, and electrode coatings (capable of application through electroplating) include, but are not restricted to: polyimide, polyether ketone, polytetrafluoroethylene, acrylonitrile butadiene styrene, polyetherimide, polyethylene terephthalate, polycarbonate, polyamide-imide, poly(methyl methacrylate), polyphenylene sulfide, polyimide, polybutylene terephthalate, polysulfone, polyvinylidene fluoride, fluorinated ethylene propylene, polychlorotrifluoroethylene, polyamide nylons, ethylene tetrafluoroethylene, high-density polyethylene, ethylene, polyepoxides, polyorganosiloxanes, and chloro-trifluoroethylene.

In addition to or instead of thin noble metal films, other materials that may be used to create electrodes according to various embodiments include copper, mercury, graphite, and glassy carbon.

Embodiment biosensing chips for use in a transdermal sensing and analysis device may be manufactured using materials and equipment commonly used in the micro-fabrication and bio-sensing industries. Formation of conductive material layers/coatings on substrates may be accomplished using any of the various deposition techniques known in the art. Substrate layers may be made of a variety of materials such as glass, plastic, metal or silicon. Polyimide films in the various embodiments may be, for example, those marketed as Apical® by Kaneka Corp., as Kapton® by Dupont™, and/or others.

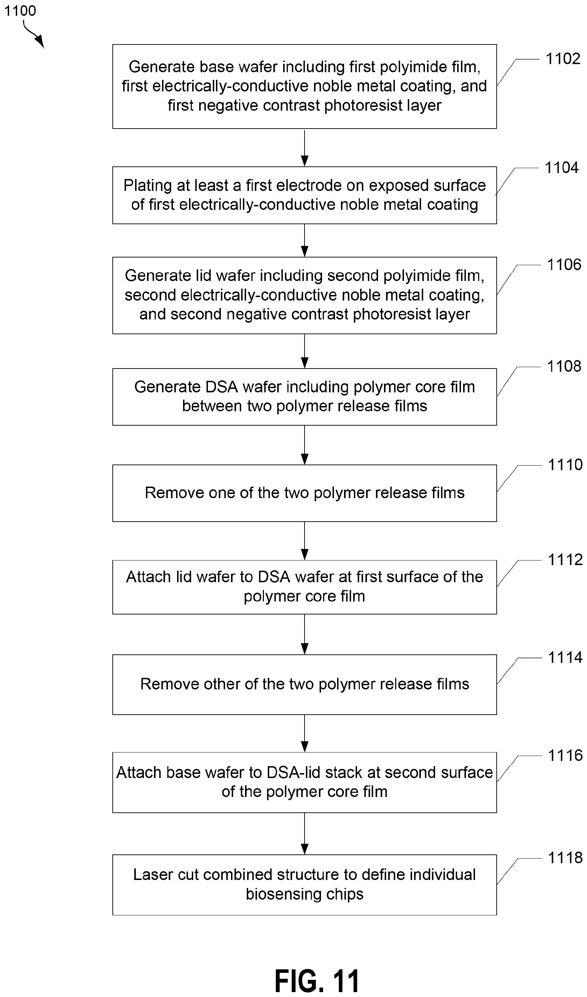

FIG. 11 is a process flow diagram illustrating an example method 1100 for manufacturing a plurality of biosensing chips. Deposition of the various layers and coatings in method 1100 may be accomplished using any of a variety of techniques known in the art. Such techniques may include, but are not limited to, lamination, sputter deposition, vacuum deposition, ion plating, ion beam assisted deposition (IBAD), pulsed laser deposition, spin coating, electroplating, electroless plating, spraying, etc.

In step 1102, a base wafer including a first polyimide film, a first electrically-conductive noble metal coating, and a first negative contrast photoresist layer may be formed. As described, in some embodiments the electrically-conductive noble metal coating may be gold, and the negative contrast photoresist layer may be an epoxy-based material (e.g., KMPR®).

In step 1104, at least a first electrode may be plated on an exposed surface of the first electrically-conductive noble metal coating.

In step 1106, a lid wafer including a second polyimide film, a second electrically-conductive noble metal coating, and a second negative contrast photoresist layer may be formed. In step 1106, at least a second electrode may be fabricated by selectively applying a sensing hydrogel to an exposed surface of the second electrically-conductive noble metal coating.

In step 1108, a DSA wafer may be formed that includes a polymer core film between two polymer release films. In various embodiments, two surfaces of the polymer core film may be coated with an acrylic adhesive.

In step 1110, one of the two polymer release films may be removed, exposing the acrylic adhesive on a first surface of the polymer core film. In step 1112, the lid wafer may be attached to the DSA wafer at the first surface of the polymer core film. In this manner, a DSA-lid stack may be formed.

In step 1114, the other of the two polymer release films may be removed, exposing the acrylic adhesive on a second surface of the polymer core film. In step 1116, the base wafer may be attached to the DSA-lid stack at the second surface of the polymer core film. In this manner, a combined structure may be created.

In step 1118, the combined structure may be laser cut to define individual biosensing chips.

Various embodiments may include example transdermal sampling and analysis devices as described below. Various embodiments may include methods of manufacturing a plurality of biosensing chips as described below.

Example 1. A transdermal sampling and analysis device comprising: a base structure comprising at least one disruptor mounted on a base substrate, wherein the at least one disruptor is configured to generate a localized heat capable of altering permeability characteristics of a subject's skin, and a first electrode array; a lid structure comprising a lid substrate having at least one hole configured to align with a portion of the at least one disruptor, wherein a region of the subject's skin is exposed to the portion of the at least one disruptor on contact with the lid substrate, and a second electrode array; an adhesive structure comprising a core substrate coated with a thin continuous adhesive film on each external surface, wherein the adhesive structure has at least a first hole configured to direct interstitial fluid collected from the subject's skin to at least one of the first electrode array and the second electrode array; at least one electrically conductive material passing through at least a second hole in the adhesive structure, wherein the at least one electrically conductive material provides a conductive path between the base structure and the lid structure; and at least one conductive trace configured for external connection to one or more of a voltage/current source and a current detector.

Example 2. The transdermal sampling and analysis device of example 1, wherein the first electrode array comprises at least one combined counter/reference electrode.

Example 3. The transdermal sensing and analysis device of example 1, wherein the second electrode array comprises at least one working electrode.

Example 4. The transdermal sensing and analysis device of example 3, wherein the at least one working electrode comprises a plurality of working electrodes each including a sensing layer configured to detect a distinct analyte.

Example 5. The transdermal sampling and analysis device of example 4, wherein the sensing layer of at least one of the plurality of working electrodes comprises at least one cofactor.

Example 6. The transdermal sampling and analysis device of example 4, wherein at least one of the plurality of working electrodes is coated with an anti-interferent barrier layer.

Example 7. The transdermal sampling and analysis device of example 6, wherein the anti-interferent barrier layer comprises alginate.

Example 8. The transdermal sampling and analysis device of example 4, wherein each sensing layer comprises an enzyme immobilized within a hydrogel, wherein the enzyme causes a reaction to determine levels of a target analyte in the collected interstitial fluid.

Example 9. The transdermal sampling and analysis device of example 4, wherein the plurality of working electrodes comprises a first working electrode configured to detect glucose, and a second working electrode configured to detect an analyte selected from alcohol or lactate.

Example 10. The transdermal sampling and analysis device of example 4, wherein the plurality of working electrodes includes: a first working electrode comprising a glucose oxidase sensing layer; and a second working electrode comprising an oxidoreductase sensing layer.

Example 11. The transdermal sampling and analysis device of example 10, wherein the oxidoreductase is selected from alcohol dehydrogenases or lactate dehydrogenases.

Example 12. The transdermal sampling and analysis device of example 1, wherein the base structure and the lid structure are aligned such that the first electrode array is positioned directly opposite the second electrode array.

Example 13. The transdermal sampling and analysis device of example 1, wherein the at least one disruptor has a serpentine configuration.

Example 14. A method of manufacturing a plurality of biosensing chips, the method comprising: generating a base wafer comprising a first polyimide film, a first electrically-conductive noble metal coating, and a first negative contrast photoresist layer; plating at least a first electrode on an exposed surface of the first electrically-conductive noble metal coating; generating a lid wafer comprising a second polyimide film, a second electrically-conductive noble metal coating, and a second negative contrast photoresist layer; fabricating at least a second electrode by selectively applying a sensing hydrogel to an exposed surface of the second electrically-conductive noble metal coating; generating a dual-sided adhesive (DSA) wafer comprising a polymer core film between two polymer release films, wherein the polymer core film is coated with an acrylic adhesive on two surfaces; removing one of the two polymer release films, wherein the acrylic adhesive on a first surface of the polymer core film is exposed; attaching the lid wafer to the DSA layer at the first surface of the polymer core film, wherein a DSA-lid stack is created; removing the other of the two polymer release films, wherein the acrylic adhesive on a second surface of the polymer core film is exposed; attaching the base wafer to the DSA-lid stack at the second surface of the polymer core film, wherein a combined structure is created; and laser cutting the combined structure to define individual biosensing chips.

Example 15. The method of example 14, further comprising depositing a conductive material onto at least one area of the base wafer, wherein the deposited conductive material electrically connects the first and second electrically conductive noble metal coatings in the combined structure.

Example 16. The method of example 14, wherein fabricating at least a second electrode comprises fabricating a plurality of working electrodes, wherein each sensing hydrogel of the plurality of working electrodes comprises a distinct reagent configured to detect a target analyte within a biological fluid sample.

Example 17. The method of example 16, wherein the plurality of working electrodes includes at least a first working electrode configured to detect glucose, and at least a second working electrode configured to detect an analyte selected from alcohol or lactate.

Example 18. The method of example 14, wherein the first electrically-conductive noble metal coating and the second electrically-conductive noble metal coating each comprise gold.

Example 19. The method of example 14, wherein plating at least a first electrode on the exposed surface of the first electrically-conductive noble metal coating comprises: plating one or more region of pure silver; and electrochemically corroding a portion of the pure silver to make silver chloride.

Example 20. The method of example 14, wherein the first and second negative contrast photoresist layers each comprise an epoxy-based photoresist material.

Example 21. The method of example 14, wherein the first polyimide film has a thickness of about 125 micrometers, and wherein the second polyamide film has a thickness of about 12-25 micrometers.

Example 22. The method of example 14, wherein generating the base wafer comprises depositing a first polyimide film, the first electrically-conductive noble metal coating, and the first negative contrast photoresist layer on a first glass substrate.

Example 23. The method of example 22, wherein generating the base wafer further comprises laminating a double-sided ultraviolet (UV) light release film onto the first glass substrate prior to depositing the first polyimide film, the first electrically-conductive noble metal coating, and the first negative contrast photoresist layer.

Example 24. The method of example 14, wherein generating the lid wafer comprises depositing a second polyimide film, a second electrically-conductive noble metal coating, and a second negative contrast photoresist layer on a second glass substrate.

Example 25. The method of example 24, wherein generating the base wafer further comprises laminating a double-sided ultraviolet (UV) light release film onto the second glass substrate prior to depositing the second polyimide film, the second electrically-conductive noble metal coating, and the second negative contrast photoresist layer.

The transdermal sampling and analysis devices/transdermal biosensors of the various embodiments may be manufactured using different methods and materials. Manufacturing methods for an embodiment transdermal sampling and analysis device are disclosed in the related International Application Number PCT/US2006/023194, filed June 14, 2006, entitled "Flexible Apparatus and Method for Monitoring and Delivery," which claims priority to the International Application Number PCT/US2005/044287, entitled "Apparatus and Method for Continuous Real-Time Trace Bimolecular Sampling, Analysis and Deliver," filed on December 9, 2005. The manufacture of an embodiment transdermal sampling and analysis device 100, 200, 300, is also disclosed in the publication entitled "Novel Non-Intrusive Trans-Dermal Remote Wireless Micro-Fluidic Monitoring System Applied to Continuous Glucose and Lactate Assays for Casualty and Combat Readiness Assessment" by John F. Currie, Michael M. Bodo and Frederick J. Pearce, RTO-MP-HFM-109:24-1, August 16, 2004. The entire contents of these related applications and the publication are incorporated by reference herein.

The preceding description of the disclosed aspects is provided to enable any person skilled in the art to make or use the present invention. Various modifications to these aspects will be readily apparent to those skilled in the art, and the generic principles defined herein may be applied to other aspects without departing from the scope of the invention. Thus, the present invention is not intended to be limited to the aspects shown herein but is to be accorded the widest scope consistent with the principles and novel features disclosed herein.

While the invention has been described in detail with respect to specific embodiments thereof, it will be apparent to those skilled in the art that various alterations, modifications and other changes may be made without departing from the scope of the embodiments described herein. It is therefore intended that all such modifications, alterations and other changes be encompassed by the claims. Further, any reference to claim elements in the singular, for example, using the articles "a," "an" or "the" is not to be construed as limiting the element to the singular.

**Claims**

1. A transdermal sampling and analysis device comprising:

    a base structure comprising:

    at least one disruptor mounted on a base substrate, wherein the at least one disruptor is configured to generate a localized heat capable of altering permeability characteristics of a subject's skin; and
    a first electrode array;

    a lid structure comprising:

    a lid substrate having at least one hole configured to align with a portion of the at least one disruptor, wherein a region of the subject's skin is exposed to the portion of the at least one disruptor on contact with the lid substrate; and
    a second electrode array;

    an adhesive structure comprising a core substrate coated with a thin continuous adhesive film on each external surface, wherein the adhesive structure has at least a first hole configured to direct interstitial fluid collected from the subject's skin to at least one of the first electrode array and the second electrode array;
    at least one electrically conductive material passing through at least a second hole in the adhesive structure, wherein the at least one electrically conductive material provides a conductive path between the base structure and the lid structure; and
    at least one conductive trace configured for external connection to one or more of a voltage/current source and a current detector.

2. The transdermal sensing and analysis device of claim 1, wherein the first electrode array comprises at least one combined counter/reference electrode and/or the second electrode array comprises at least one working electrode.

3. The transdermal sensing and analysis device of claim 2, wherein the at least one working electrode comprises a plurality of working electrodes each including a sensing layer configured to detect a distinct analyte.

4. The transdermal sampling and analysis device of claim 3, wherein the sensing layer of at least one of the plurality of working electrodes

    comprises at least one cofactor, and/or
    is coated with an anti-interferent barrier layer, preferably comprising alginate.

5. The transdermal sampling and analysis device according to any one of claims 3 to 4, wherein each sensing layer comprises an enzyme immobilized within a hydrogel, wherein the enzyme causes a reaction to determine levels of a target analyte in the collected interstitial fluid.

6. The transdermal sampling and analysis device according to any one of claims 3 to 5, wherein the plurality of working electrodes comprises a first working electrode configured to detect glucose, and a second working electrode configured to detect an analyte selected from alcohol or lactate.

7.  The transdermal sampling and analysis device according to any one of claims 3 to 6, wherein the plurality of working electrodes includes:

    a first working electrode comprising a glucose oxidase sensing layer; and
    a second working electrode comprising an oxidoreductase sensing layer preferably selected from alcohol dehydrogenases or lactate dehydrogenases.

8.  The transdermal sampling and analysis device according to any one of claims 1 to 7, wherein the base structure and the lid structure are aligned such that the first electrode array is positioned directly opposite the second electrode array, and/or the at least one disruptor has a serpentine configuration.

9.  A method of manufacturing a plurality of biosensing chips, the method comprising:

    forming a base wafer comprising a first polyimide film having preferably a thickness of about 125 micrometers, a first electrically-conductive noble metal coating, and a first negative contrast photoresist layer, preferably comprising an epoxy-based photoresist material;
    plating at least a first electrode on an exposed surface of the first electrically-conductive noble metal coating, preferably comprising gold;
    forming a lid wafer comprising a second polyimide film having preferably a thickness of about 12-25 micrometers, a second electrically-conductive noble metal coating, and a second negative contrast photoresist layer preferably comprising an epoxy-based photoresist material;
    fabricating at least a second electrode by selectively applying a sensing hydrogel to an exposed surface of the second electrically-conductive noble metal coating, preferably comprising gold;
    forming a dual-sided adhesive (DSA) wafer comprising a polymer core film between two polymer release films, wherein the polymer core film is coated with an acrylic adhesive on two surfaces;
    removing one of the two polymer release films, wherein the acrylic adhesive on a first surface of the polymer core film is exposed;
    attaching the lid wafer to the DSA wafer at the first surface of the polymer core film, wherein a DSA-lid stack is created;
    removing the other of the two polymer release films, wherein the acrylic adhesive on a second surface of the polymer core film is exposed;
    attaching the base wafer to the DSA-lid stack at the second surface of the polymer core film, wherein a combined structure is created; and
    laser cutting the combined structure to define individual biosensing chips.

10. The method of claim 9, further comprising depositing a conductive material onto at least one area of the base wafer, wherein the deposited conductive material electrically connects the first and second electrically conductive noble metal coatings in the combined structure.

11. The method of claim 9 or 10, wherein fabricating at least a second electrode comprises fabricating a plurality of working electrodes, wherein each sensing hydrogel of the plurality of working electrodes comprises a distinct reagent configured to detect a target analyte within a biological fluid sample.

12. The method of claim 11, wherein the plurality of working electrodes includes at least a first working electrode configured to detect glucose, and at least a second working electrode configured to detect an analyte selected from alcohol or lactate.

13. The method according to any one of claims 1 to 12, wherein plating at least a first electrode on the exposed surface of the first electrically-conductive noble metal coating comprises:

    plating one or more region of pure silver; and
    electrochemically corroding a portion of the pure silver to make silver chloride.

14. The method according to any one of claims 1 to 13, wherein generating the base wafer comprises

depositing a first polyimide film, the first electrically-conductive noble metal coating, and the first negative contrast photoresist layer on a first glass substrate, and/or

laminating a double-sided ultraviolet (UV) light release film onto the first glass substrate prior to depositing the first polyimide film, the first electrically-conductive noble metal coating, and the first negative contrast photoresist layer.

15. The method according to any one of claims 1 to 14, wherein generating the lid wafer comprises

depositing a second polyimide film, a second electrically-conductive noble metal coating, and a second negative contrast photoresist layer on a second glass substrate, and/or

laminating a double-sided ultraviolet (UV) light release film onto the second glass substrate prior to depositing the second polyimide film, the second electrically-conductive noble metal coating, and the second negative contrast photoresist layer.

**FIG. 1A**

**FIG. 1B**

200

224

212

216

214

222

218

210

204

202

206

220

208

**FIG. 2**

**FIG. 3**

FIG. 4

FIG. 5

500

508

604

514  512  510

602

# FIG. 6A

650

E4

E3

E2

E1

E6

E5

502

E7

**FIG. 6B**

700

712 · 708 · 710

706

Polyimide film

704

UV light release film

702

Glass substrate

## FIG. 7A

750

756 · 758
· 758

754

UV light release film

752

Glass substrate

## FIG. 7B

800

Chip

808    812                    808              810

806                                           806

Polyimide film                Polyimide film  804

UV light release film                         802

Glass substrate

FIG. 8

**FIG. 9**

802 — Glass substrate

804 — UV light release film

806 — Polyimide film | Polyimide film

808 — 810

756 — 812

808
756

1002

DSA-lid stack
900

712
708
710
710

706 — Polyimide film

704 — UV light release film

702 — Glass substrate

Base wafer
700

FIG. 10A

**FIG. 10B**

1100

```
┌─────────────────────────────────────────────────┐
│ Generate base wafer including first polyimide film, │──── 1102
│ first electrically-conductive noble metal coating, and │
│ first negative contrast photoresist layer         │
└─────────────────────────────────────────────────┘
                        │
                        ▼
┌─────────────────────────────────────────────────┐
│ Plating at least a first electrode on exposed surface │──── 1104
│ of first electrically-conductive noble metal coating │
└─────────────────────────────────────────────────┘
                        │
                        ▼
┌─────────────────────────────────────────────────┐
│ Generate lid wafer including second polyimide film, │──── 1106
│ second electrically-conductive noble metal coating, │
│ and second negative contrast photoresist layer   │
└─────────────────────────────────────────────────┘
                        │
                        ▼
┌─────────────────────────────────────────────────┐
│ Generate DSA wafer including polymer core film    │──── 1108
│ between two polymer release films                 │
└─────────────────────────────────────────────────┘
                        │
                        ▼
┌─────────────────────────────────────────────────┐
│ Remove one of the two polymer release films       │──── 1110
└─────────────────────────────────────────────────┘
                        │
                        ▼
┌─────────────────────────────────────────────────┐
│ Attach lid wafer to DSA wafer at first surface of the │──── 1112
│ polymer core film                                 │
└─────────────────────────────────────────────────┘
                        │
                        ▼
┌─────────────────────────────────────────────────┐
│ Remove other of the two polymer release films     │──── 1114
└─────────────────────────────────────────────────┘
                        │
                        ▼
┌─────────────────────────────────────────────────┐
│ Attach base wafer to DSA-lid stack at second surface │──── 1116
│ of the polymer core film                          │
└─────────────────────────────────────────────────┘
                        │
                        ▼
┌─────────────────────────────────────────────────┐
│ Laser cut combined structure to define individual │──── 1118
│ biosensing chips                                  │
└─────────────────────────────────────────────────┘
```

# FIG. 11

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/267501 A1 (JACHMANN EMIL F [US] ET AL) 2 September 2021 (2021-09-02)<br>* figures 1B,2,3 *<br>* paragraphs [0038] - [0040], [0047] - [0048], [0057] - [0066], [0081] * | 1-15 | INV.<br>A61B5/145<br>A61B5/1477<br>A61B5/1486<br>A61B5/15<br>A61B5/00 |
| A | WO 2007/070093 A2 (FLEXIBLE MEDICAL SYSTEMS LLC [US]; CURRIE JOHN FREDERICK [US] ET AL.) 21 June 2007 (2007-06-21)<br>* paragraph [0115] * | 9 | |
| A | US 5 466 575 A (COZZETTE STEPHEN N [US] ET AL) 14 November 1995 (1995-11-14)<br>* column 66, lines 8-57 * | 13 | |

**TECHNICAL FIELDS SEARCHED (IPC)**

A61B

The present search report has been drawn up for all claims

| Place of search | Date of completion of the search | Examiner |
|---|---|---|
| The Hague | 21 May 2025 | Oancea, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 613 193 A1

## ANNEX TO THE EUROPEAN SEARCH REPORT
## ON EUROPEAN PATENT APPLICATION NO.

EP 25 16 1525

This annex lists the patent family members relating to the patent documents cited in the above-mentioned European search report.
The members are as contained in the European Patent Office EDP file on
The European Patent Office is in no way liable for these particulars which are merely given for the purpose of information.

21-05-2025

| Patent document cited in search report | | Publication date | Patent family member(s) | | Publication date |
|---|---|---|---|---|---|
| US 2021267501 | A1 | 02-09-2021 | CN 115334967 | A | 11-11-2022 |
| | | | US 2021267501 | A1 | 02-09-2021 |
| | | | WO 2021173210 | A1 | 02-09-2021 |
| WO 2007070093 | A2 | 21-06-2007 | JP 2009518113 | A | 07-05-2009 |
| | | | WO 2007070093 | A2 | 21-06-2007 |
| US 5466575 | A | 14-11-1995 | AT E213833 | T1 | 15-03-2002 |
| | | | CA 2002848 | A1 | 14-05-1990 |
| | | | CA 2221178 | A1 | 14-05-1990 |
| | | | DE 68929373 | T2 | 10-10-2002 |
| | | | EP 0442969 | A1 | 28-08-1991 |
| | | | JP 3105919 | B2 | 06-11-2000 |
| | | | JP 3137612 | B2 | 26-02-2001 |
| | | | JP H04503249 | A | 11-06-1992 |
| | | | JP 2000065791 | A | 03-03-2000 |
| | | | KR 900702361 | A | 06-12-1990 |
| | | | TW 219975 | B | 01-02-1994 |
| | | | US 5200051 | A | 06-04-1993 |
| | | | US 5466575 | A | 14-11-1995 |
| | | | US 5554339 | A | 10-09-1996 |
| | | | US 5837446 | A | 17-11-1998 |
| | | | US 5837454 | A | 17-11-1998 |
| | | | WO 9005910 | A1 | 31-05-1990 |

EPO FORM P0459

For more details about this annex : see Official Journal of the European Patent Office, No. 12/82

**Patent documents cited in the description**

- US 59417524
- US 9877673 B
- US 208344
- WO 2007070093 A
- US 8364228 B
- US 2006023194 W
- US 2005044287 W

**Non-patent literature cited in the description**

- **JOHN F. CURRIE** ; **MICHAEL M. BODO** ; **FREDER-ICK J. PEARCE**. Novel Non-Intrusive Trans-Dermal Remote Wireless Micro-Fluidic Monitoring System Applied to Continuous Glucose and Lactate Assays for Casualty and Combat Readiness Assessment. *RTO-MP-HFM-109:24-1*, 16 August 2004